# EUROPEAN PATENT SPECIFICATION

(11) **EP 2 421 865 B1**
(45) Date of publication and mention of the grant of the patent: **10.07.2013**
(21) Application number: 10713606.1
(22) Date of filing: 30.03.2010
(51) Int. Cl.: C07D 471/10, A01N 43/90

(54) **SPIROHETEROCYCLIC PYRROLIDINE DIONE DERIVATIVES USED AS PESTICIDES**
ALS PESTIZIDE VERWENDETE SPIROHETEROCYCLISCHE PYRROLIDINDIONDERIVATE
DÉRIVÉS SPIRO-HÉTÉROCYCLIQUES DE PYRROLIDINE-DIONE UTILISÉS COMME PESTICIDES

(30) Priority: 08.04.2009 GB 0906164
(43) Date of publication of application: 29.02.2012
(73) Proprietor: Syngenta Participations AG, 4332 Stein (CH)
(72) Inventor: MUEHLEBACH, Michel, CH-4332 Stein (CH); HUETER, Ottmar Franz, CH-4332 Stein (CH)
(74) Representative: Herrmann, Jörg
(86) International application number: PCT/EP2010/054216
(87) International publication number: WO 2010/115780

(56) References cited:
- WO-A1-2009/049851
- US-B2- 6 774 133

## Description

The present invention relates to new 3-substituted spiroheterocyclic pyrrolidine dione derivatives, to processes for preparing them, to pesticidal, in particular insecticidal, acaricidal, molluscicidal and nematicidal compositions comprising them and to methods of using them to combat and control pests such as insect, acarine, mollusc and nematode pests.

Spiroheterocyclic pyrrolidine dione derivatives are disclosed for example in US 6555567, US 6479489, US 6774133, EP 596298, WO 98/05638 and WO 99/48869. Further, spiroheterocyclic pyrrolidine dione derivatives are known, for example, from WO 09/049851.

It has now surprisingly been found that certain novel 3-substituted spiroheterocyclic pyrrolidine dione derivatives have good insecticidal properties.

The present invention therefore provides compounds of formula I 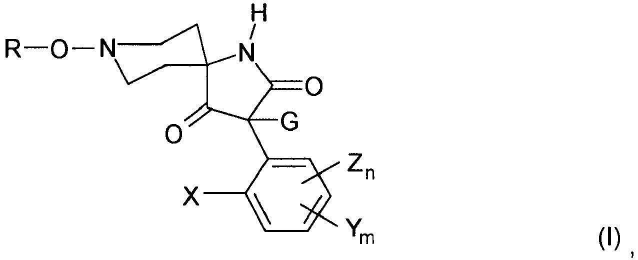 wherein
X , Y and Z, independently of each other, are C₁₋₄alkyl, C₃₋₆cycloalkyl, C₁₋₄haloalkyl, C₁₋₄alkoxy, halogen, phenyl or phenyl substituted by C₁₋₄alkyl, C₁₋₄haloalkyl, halogen or cyano;
m and n, independently of each other, are 0, 1, 2 or 3, where m+n is 0, 1, 2 or 3;
G is halogen or nitro; and
R is hydrogen, C₁₋₆alkyl, C₁₋₆haloalkyl, C₁₋₆cyanoalkyl, C₂-C₆alkenyl, C₃-C₆alkynyl, benzyl, C₁₋₄alkoxy(C₁₋₄)alkyl or C₁₋₄alkoxy(C₁₋₄)alkoxy(C₁₋₄)alkyl; or
an agrochemically acceptable salt or an N-oxide thereof.

In the compounds of the formula I, each alkyl moiety either alone or as part of a larger group is a straight or branched chain and is, for example, methyl, ethyl, n-propyl, n-butyl, iso-propyl, sec-butyl, iso-butyl, tert-butyl, n-pentyl, iso-pentyl and n-hexyl.

Alkoxy groups preferably have a preferred chain length of from 1 to 4 carbon atoms. Alkoxy is, for example, methoxy, ethoxy, propoxy, i-propoxy, n-butoxy, isobutoxy, sec-butoxy and tert-butoxy. Such groups can be part of a larger group such as alkoxyalkyl and alkoxyalkoxyalkyl. Alkoxyalkyl groups preferably have a chain length of 1 to 4 carbon atoms. Alkoxyalkyl is, for example, methoxymethyl, methoxyethyl, ethoxymethyl, ethoxyethyl, n-propoxymethyl, n-propoxyethyl or isopropoxymethyl.

Halogen is generally fluorine, chlorine, bromine or iodine. This also applies, correspondingly, to halogen in combination with other meanings, such as haloalkyl.

Haloalkyl groups preferably have a chain length of from 1 to 6 carbon atoms. Haloalkyl is, for example, fluoromethyl, difluoromethyl, trifluoromethyl, chloromethyl, dichloromethyl, trichloromethyl, 2,2,2-trifluoroethyl, 2-fluoroethyl, 2-chloroethyl, pentafluoroethyl, 1,1-difluoro-2,2,2-trichloroethyl, 2,2,3,3-tetrafluoroethyl and 2,2,2-trichloroethyl; preferably trichloromethyl, difluorochloromethyl, difluoromethyl, trifluoromethyl and dichlorofluoromethyl.

The cycloalkyl groups preferably have from 3 to 6 ring carbon atoms, for example cyclopropyl, cyclobutyl, cyclopentyl and cyclohexyl.

Phenyl, , may be substituted, preferably by C₁-₄alkyl, C₁₋₄haloalkyl, halogen or cyano groups . In this case, the substituents can be in ortho, meta and/or para position. The preferred substituent positions are the ortho and para positions to the ring attachment point.

The group G is a latentiating group which is selected to allow its removal by one or a combination of biochemical, chemical or physical processes to afford compounds of formula I where G is hydrogen before, during or following application to the treated area or plants. Examples of these processes include enzymatic cleavage, chemical hydrolysis and photoloysis. Compounds bearing such groups G may offer certain advantages, such as improved penetration of the cuticula of the plants treated, increased tolerance of crops, improved compatibility or stability in formulated mixtures containing other herbicides, improved compatibility or stability in formulated mixtures containing other herbicides, herbicide safeners, plant growth regulators, fungicides or insecticides, or reduced leaching in soils.

The compounds of formula I may exist in different geometric or optical isomers or different tautomeric forms. One or more centres of chirality may be present, in which case compounds of the formula I may be present as pure enantiomers, mixtures of enantiomers, pure diastereomers or mixtures of diastereomers. Centres of tautomerisation may be present. This invention covers all such isomers and tautomers and mixtures thereof in all proportions as well as isotopic forms such as deuterated compounds.

The invention relates also to the agriculturally acceptable salts which the compounds of formula I are able to form with transition metal, alkali metal and alkaline earth metal bases, amines, quaternary ammonium bases or tertiary sulfonium bases.

Among the transition metal, alkali metal and alkaline earth metal salt formers, special mention should be made of the hydroxides of copper, iron, lithium, sodium, potassium, magnesium and calcium, and preferably the hydroxides, bicarbonates and carbonates of sodium and potassium.

Examples of amines suitable for ammonium salt formation include ammonia as well as primary, secondary and tertiary C₁-C₁₈alkylamines, C₁-C₄hydroxyalkylamines and C₂-C₄alkoxyalkyl-amines, for example methylamine, ethylamine, n-propylamine, *i-*propylamine, the four butylamine isomers, n-amylamine, *i*-amylamine, hexylamine, heptylamine, octylamine, nonylamine, decylamine, pentadecylamine, hexadecylamine, heptadecylamine, octadecylamine, methylethylamine, methylisopropylamine, methylhexylamine, methylnonylamine, methylpentadecylamine, methyloctadecylamine, ethylbutylamine, ethylheptylamine, ethyloctylamine, hexylheptylamine, hexyloctylamine, dimethylamine, diethylamine, di-*n*-propylamine, di-*i*-propylamine, di-n-butylamine, di-*n-*amylamine, di-*i*-amylamine, dihexylamine, diheptylamine, dioctylamine, ethanolamine, *n-*propanolamine, *i*-propanolamine, *N,N-*diethanolamine, *N*-ethylpropanolamine, *N-*butylethanolamine, allylamine, *n*-but-2-enylamine, *n*-pent-2-enylamine, 2,3-dimethylbut-2-enylamine, dibut-2-enylamine, *n*-hex-2-enylamine, propylenediamine, trimethylamine, triethylamine, tri-*n*-propylamine, tri-*i*-opropylamine, tri-*n*-butylamine, tri-*i*-butylamine, tri-sec-butylamine, tri-*n*-amylamine, methoxyethylamine and ethoxyethylamine; heterocyclic amines, for example pyridine, quinoline, isoquinoline, morpholine, piperidine, pyrrolidine, indoline, quinuclidine and azepine; primary arylamines, for example anilines, methoxyanilines, ethoxyanilines, *o*-, m- and *p*-toluidines, phenylenediamines, benzidines, naphthylamines and *o*-, *m*- and *p*-chloroanilines; but especially triethylamine, *i*-propylamine and di-*i*-propylamine.

Preferred quaternary ammonium bases suitable for salt formation correspond, for example, to the formula [N(Rₐ R_{b} R_{c} R_{d})]OH, wherein Rₐ, R_{b}, R_{c} and R_{d} are each independently of the others hydrogen or C₁-C₄alkyl. Further suitable tetraalkylammonium bases with other anions can be obtained, for example, by anion exchange reactions.

Preferred tertiary sulfonium bases suitable for salt formation correspond, for example, to the formula [SRₑR_{f}R_{g}]OH, wherein Rₑ, R_{f} and R_{g} are each independently of the others C₁-C₄ alkyl. Trimethylsulfonium hydroxide is especially preferred. Suitable sulfonium bases may be obtained from the reaction of thioethers, in particular dialkylsulfides, with alkylhalides, followed by conversion to a suitable base, for example a hydroxide, by anion exchange reactions.

The compounds of formula I according to the invention also include hydrates which may be formed during the salt formation.

The preferred values of G, R, X, Y, Z, m and n in the compounds of formula I in any combination thereof are set out below.

Preferably G is fluoro, chloro, bromo, iodo or nitro. It is preferred that G is fluoro, chloro, bromo or nitro and even more preferably G is fluoro, chloro or nitro.

Preferably R is hydrogen, C₁₋₆alkyl, C₁₋₆haloalkyl, C₁₋₆cyanoalkyl, C₂-C₆alkenyl, C₃-C₆alkynyl, benzyl, C₁₋₄alkoxy(C₁₋₄)alkyl or C₁₋₄alkoxy(C₁₋₄)alkoxy(C₁₋₄)alkyl, in particular hydrogen, methyl, ethyl, n-propyl, isopropyl, cyanomethyl, trifluoromethyl, 2,2,2-trifluoroethyl, allyl, propargyl, benzyl, methoxymethyl, ethoxymethyl, methoxyethyl or methoxyethoxymethyl.

Preferably X is C₁-C₄alkyl, C₃-C₆cycloalkyl, C₁-C₄alkoxy or halogen.

More preferably X is methyl, ethyl, cyclopropyl, methoxy, fluoro, bromo or chloro.

Preferably Y and Z, independently of each other, are C₁-C₄alkyl, C₃-C₆cycloalkyl, C₁-C₄alkoxy, halogen, phenyl, phenyl substituted by C₁-C₄alkyl or halo-substituted phenyl and m+n is 1, 2 or 3 and in particular m+n is 1 or 2.

More preferably Y and Z, independently of each other, are methyl, ethyl, cyclopropyl, methoxy, fluoro, bromo or chloro, phenyl or halo-substituted phenyl (in particular fluorophenyl or chlorophenyl and especially 4-chlorophenyl or 4-fluorophenyl) and m+n is 1, 2 or 3 and in particular m+n is 1 or 2.

Yet more preferably Y and Z are each independently methyl, ethyl, cyclopropyl, methoxy, fluoro, bromo or chloro and m+n is 1, 2 or 3, in particular, m+n is 1 or 2.

In one preferred group of compounds of the formula (I), R is hydrogen, methyl, ethyl, 2,2,2-trifluoroethyl, allyl, propargyl, benzyl, methoxymethyl, ethoxymethyl, methoxyethyl or methoxyethoxymethyl, X is methyl, ethyl, cyclopropyl, methoxy, fluoro, bromo or chloro, Y and Z, independently of each other, are methyl, ethyl, cyclopropyl, methoxy, fluoro, chloro, bromo, phenyl or phenyl substituted by halogen or C₁-C₂alkyl, G has the meanings assigned to it above and m+n is 1, 2 or 3.

In another preferred group of compounds of the formula (I), R is methyl, ethyl, 2,2,2-trifluoroethyl, allyl, propargyl, benzyl or methoxymethyl, X is methyl, ethyl, cyclopropyl, methoxy, fluoro, bromo or chloro, Y and Z, independently of each other, are methyl, ethyl, cyclopropyl, methoxy, fluoro, chloro, bromo, phenyl or phenyl substituted by halogen or C₁-C₂alkyl, G has the meanings assigned to it above and m±n is 1, 2 or 3.

In yet another preferred group of compounds of the formula (I), R is methyl, ethyl, 2,2,2-trifluoroethyl, benzyl or methoxymethyl, X is methyl, ethyl, cyclopropyl, methoxy, fluoro, bromo or chloro, Y and Z, independently of each other, are methyl, ethyl, cyclopropyl, methoxy, fluoro, chloro, bromo, phenyl or phenyl substituted by halogen or C₁-C₂alkyl, G is fluoro, chloro or nitro and m+n is 1-3.

The invention covers also salts of the compounds of the formula I with amines, alkali metal and alkaline earth metal bases or quaternary ammonium bases.

Among the alkali metal and alkaline earth metal hydroxides as salt formers, special mention should be made of the hydroxides of lithium, sodium, potassium, magnesium and calcium, but especially the hydroxides of sodium and potassium. The compounds of formula I according to the invention also include hydrates which may be formed during the salt formation.

Examples of amines suitable for ammonium salt formation include ammonia as well as primary, secondary and tertiary C₁-C₁₈alkylamines, C₁-C₄hydroxyalkylamines and C₂-C₄alkoxyalkylamines, for example methylamine, ethylamine, n-propylamine, isopropylamine, the four butylamine isomers, n-amylamine, isoamylamine, hexylamine, heptylamine, octylamine, nonylamine, decylamine, pentadecylamine, hexadecylamine, heptadecylamine, octadecylamine, methylethylamine, methylisopropylamine, methylhexylamine, methylnonylamine, methylpentadecylamine, methyloctadecylamine, ethylbutylamine, ethylheptylamine, ethyloctylamine, hexylheptylamine, hexyloctylamine, dimethylamine, diethylamine, di-n-propylamine, diisopropylamine, di-n-butylamine, di-n-amylamine, diisoamylamine, dihexylamine, diheptylamine, dioctylamine, ethanolamine, n-propanolamine, isopropanolamine, N,N-diethanolamine, N-ethylpropanolamine, N-butylethanolamine, allylamine, n-but-2-enylamine, n-pent-2-enylamine, 2,3-dimethylbut-2-enylamine, dibut-2-enylamine, n-hex-2-enylamine, propylenediamine, trimethylamine, triethylamine, tri-n-propylamine, triisopropylamine, tri-n-butylamine, triisobutylamine, tri-sec-butylamine, tri-n-amylamine, methoxyethylamine and ethoxyethylamine; heterocyclic amines, for example pyridine, quinoline, isoquinoline, morpholine, piperidine, pyrrolidine, indoline, quinuclidine and azepine; primary arylamines, for example anilines, methoxyanilines, ethoxyanilines, o-, m- and p-toluidines, phenylenediamines, benzidines, naphthylamines and o-, m- and p-chloroanilines; but especially triethylamine, isopropylamine and diisopropylamine. Preferred quaternary ammonium has bases suitable for salt formation correspond, for example, to the formula [N(Rₐ R_{b} R_{c} R_{d})]OH wherein Rₐ, R_{b}, R_{c} and R_{d} are each independently of the others C₁-C₄alkyl. Further suitable tetraalkylammonium bases with other anions can be obtained, for example, by anion exchange reactions.

The compounds of the invention may be made by a variety of methods. For example, the compounds of formula I, wherein the substituents have the meanings assigned to them above, can be prepared by means of processes known *per se*, e.g. by treating compounds of formula II with a halogenating or nitrating agent:

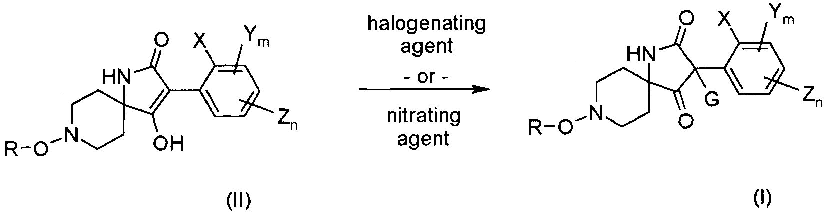

Compounds of the formula I, in which X, Y, Z, m, n and R are as defined above and wherein G is a halogen, may be prepared by known procedures as described, for example, in JP 2000086628. Typically, compounds of formula II, in which X, Y, Z, m, n and R are as defined above, are treated with halogenating agents in the presence of a solvent, optionally in the presence of a base, and, if appropriate, in the presence of a free-radical initiator. Suitable halogenating agents are, for example, chlorine, sulfuryl chloride, sulfuryl bromide, thionyl chloride, thionyl chloride, imides such as, for example, N-chlorosuccinimide or N-bromosuccinimide, chlorosulfonic acid, hypochlorites such as, for example, *tert*-butyl hypochlorite or sodium hypochlorite, electrophilic fluorinating agents such as, for example, Selectfluor [1-chloromethyl-4-fluoro-1,4-diazoniabicyclo[2.2.2]octane bis(tetrafluoroborate)], Accufluor [1-fluoro-4-hydroxy-1,4-diazoniabicyclo[2.2.2]octane bis(tetrafluoroborate)], Florinate FP-T 500 [N-fluoropyridinium trifluoromethanesulfonate] or NFSI [N-fluorodibenzenesulfonimide]. Suitable solvents for this reaction are selected to be compatible with the reagents and include, for example, carbon tetrachloride, chloroform, dichloromethane, dichloroethane, acetonitrile, ethyl acetate, benzene, toluene, chlorobenzene, tetrahydrofuran or dioxane. Suitable free-radical initiators are, for example, benzoyl peroxide, AIBN [α,α'-azodiisobutyronitrile] or the like. Suitable bases for this reaction are selected to be compatible with the reagents and include, for example, alkali metal or alkaline earth metal carbonates, acetates or hydroxides, such as sodium hydrogen carbonate, potassium carbonate, sodium acetate or lithium hydroxide.

Compounds of the formula I, in which X, Y, Z, m, n and R are as defined above and wherein G represents nitro, may be prepared by reacting compounds of formula II, in which X, Y, Z, m, n and R are as defined above, with nitrating agents, such as, for example, fuming nitric acid or nitrating acid mixtures in the presence of a solvent, preferably chloroform, dichloromethane, dichloroethane, chlorobenzene or acetic acid, under conditions known to a person skilled in the art (see for example: Nitrobarbituric Acid, W. W. Hartman, O. E. Sheppard, Organic Syntheses 12, 1932).

Depending on the nature of the substituents, compounds of formula II may exist in different isomeric forms, in particular in different tautomeric forms:

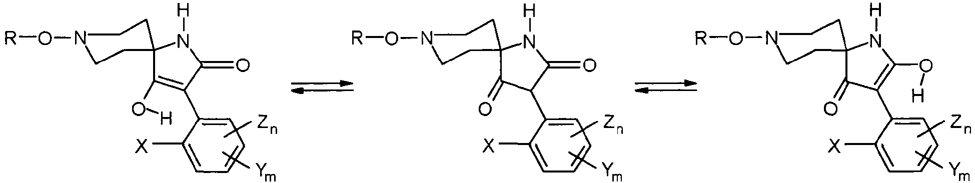

This invention covers all such isomers and tautomers and mixtures thereof in all proportions.

Compounds of formula III, in which X, Y, Z, m, n and G are as defined above, 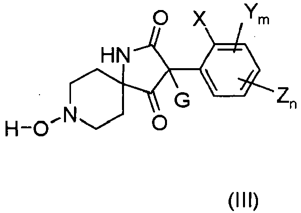 can be obtained by catalytic hydrogenation of compounds of formula I, in which X, Y, Z, m, n and G are as defined above and in which R is represented by a benzyl group.

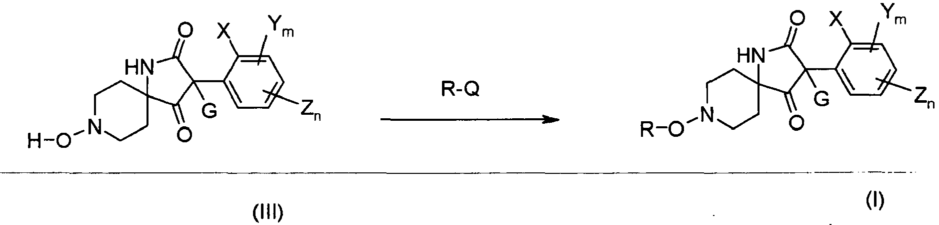

Compounds of formula I, in which X, Y, Z, m, n, R and G are as defined above, can be obtained by treating compounds of formula III, in which X, Y, Z, m, n and G are as defined above, with an alkylating agent R-Q, wherein R represents the alkyl group to be incorporated and Q represents a nucleofuge, in the presence of at least one equivalent of a base, and optionally in the presence of a suitable solvent.

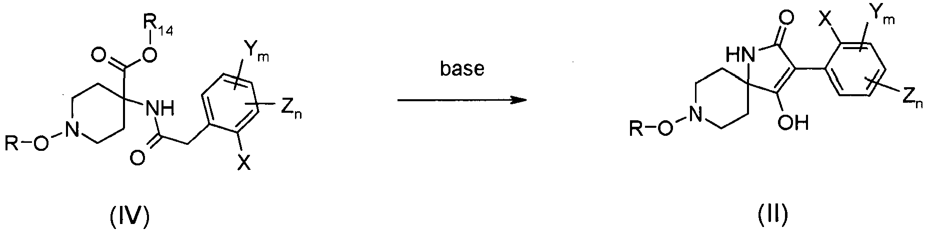

Compounds of formula II may be prepared via the cyclisation of compounds of formula IV, wherein R₁₄ is C₁₋₆alkyl, preferably in the presence of base, and optionally in the presence of a suitable solvent, by methods described, for example, in US 6774133, US 6555567 and US 6479489. X, Y, Z, m, n and R are as defined above.

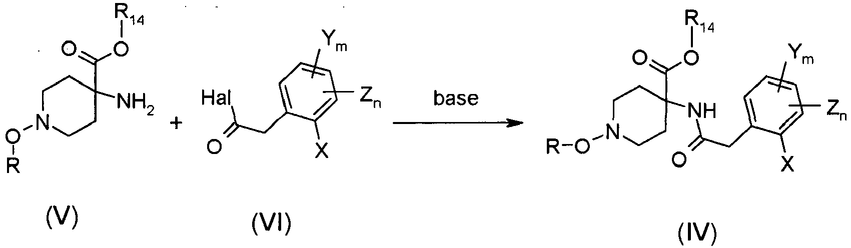

Compounds of formula IV may be prepared by reacting amino acid derivatives of formula V with phenylacetyl halides of formula VI, preferably in the presence of base in a suitable solvent by known methods described, for example, in US 6774133, US 6555567 and US 6479489. X, Y, Z, m, n, R and R₁₄ are as defined above. The base may be inorganic such as an alkali metal carbonate or hydroxide or a metal hydride, or an organic base such as a tertiary amine or metal alkoxide. Examples of suitable inorganic bases include sodium carbonate, sodium or potassium hydroxide, sodium hydride, and suitable organic bases include trialkylamines such as trimethylamine and triethylamine; pyridines or other amine bases such as 1,4-diazobicyclo[2.2.2]octane and 1,8-diazabicyclo[5.4.0]undec-7-ene. Preferred bases include triethylamine and pyridine. Suitable solvents for this reaction are selected to be compatible with the reagents and include ethers such as tetrahydrofuran and 1,2-dimethoxyethane and halogenated solvents such as dichloromethane and chloroform. Certain bases, such as pyridine and triethylamine, may be employed successfully as both base and solvent.

Phenylacetyl halides of formula VI, wherein Hal is Cl or Br and in which X, Y, Z, m, n are as defined above, are known compounds or can be prepared by known methods as described, for example, in US 6774133, US 6555567 and US 6479489.

Amino acid esters of formula V, wherein R₁₄ is C₁-C₆alkyl, can be prepared by known methods from amino acids of formula VII. Esterification of VII with an alcohol of the formula R₁₄OH under thionyl chloride activation is a typical example for the preparation of esters V, but other known esterification methods may also be applied, like for example treatment of a compound of the formula VII with an alcohol of the formula R₁₄OH under acidic conditions (typically H₂SO₄ or HCl). For the particular situation where R₁₄ is methyl, a compound of the formula VII may also be treated with diazomethane or trimethylsilyldiazomethane, or with acetyl chloride in methanol. R is as defined above.

Alternatively, amino acid esters of formula V, wherein R₁₄ is C₁-C₆alkyl, may be prepared by reacting amino nitriles of the formula VIII with an alcohol of the formula R₁₄OH, preferably in the presence of a strong acid (especially sulfuric acid or hydrochloric acid), under known conditions. R is as defined above.

The compounds VIII, VII and V can be reacted and/or isolated as free amines or amine salts (eg a hydrohalide salt, more specifically a hydrochloride or hydrobromide salt, or any other equivalent salt).

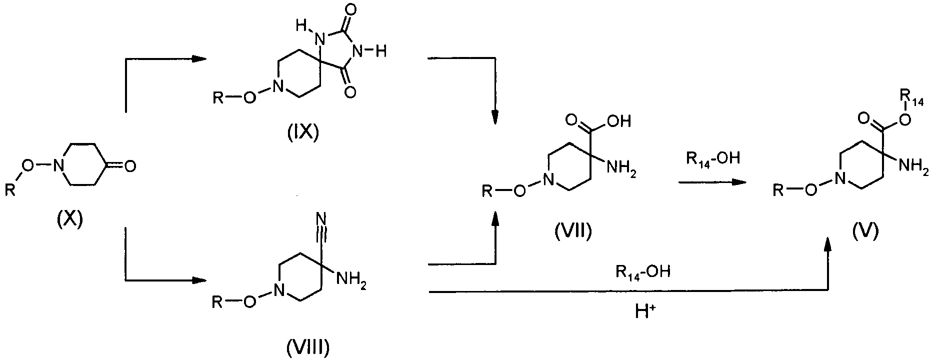

Amino acids of formula VII can themselves be prepared by known methods, typically under hydrolysis conditions usually either acidic or basic, from amino nitriles of the formula VIII. A representative example for the nitrile hydrolysis into its corresponding carboxylic acid functionality under aqueous H₂SO₄ or HCl conditions may be found, for example, in E.F.G. Duynstee et al., Recueil Trav. Chim. Pays-Bas 84, 1442-1451, (1965) or in B. Wang et al., Eur. J. Org. Chem. (2008), (2), 350-355. R is as defined above.

Ultimately, amino acids of formula VII can be prepared from ketones of formula X by means of a Strecker-type synthesis via amino nitriles of formula VIII. The transformation ketone X to amino nitriles VIII (Strecker reaction) is a well described one-pot three components coupling involving, besides ketones X, hydrogen cyanide HCN or various alkali cyanides (eg KCN, NaCN, etc.) in buffered aqueous media or trimethylsilyl cyanide TMSCN, optionally in presence of a catalytic amount of a Lewis acid, for example Znl₂, and ammonia or an ammonium salt (or their equivalents). An appropriate source of cyanide (eg HCN) may also be added to a preformed ketimine (or iminium salt) from ketone X, or a salt thereof. A summary on the scope of the Strecker reaction may be found, for example, in L. Kürti, B. Czakó, 'Strategic Applications of Named Reactions in Organic Synthesis', Elsevier Academic Press, 2005, pp. 446-447 and 690-691.

Alternatively, amino acids of formula VII can also be prepared from ketones of formula X by means of a Bucherer Bergs reaction, described for example in Th. Wieland et al., Methoden Org. Chem. (Houben-Weyl) (1959), Bd. XI/2, 305-306, via hydantoins of formula IX. Amino acids of formula VII can be prepared by known methods, typically under thermal hydrolysis conditions usually either acidic or basic, from hydantoins IX. R is as defined above.

The compounds VIII, VII and V can be reacted and/or isolated as free amines or amine salts (eg a hydrohalide salt, more specifically a hydrochloride or hydrobromide salt, or any other equivalent salt).

Compounds of formula X, where R is C₁₋₆alkyl, C₁₋₆haloalkyl, C₁₋₆cyanoalkyl, C₂-C₆alkenyl, C₃-C₆alkynyl, C₁₋₄alkoxy(C₁₋₄)alkyl, C₁₋₄alkoxy(C₁₋₄)alkoxy(C₁₋₄)alkyl or a benzyl group, are known or can be obtained, for example, according to Major and Dursch, Journal of Organic Chemistry (1961), 26, 1867-74.

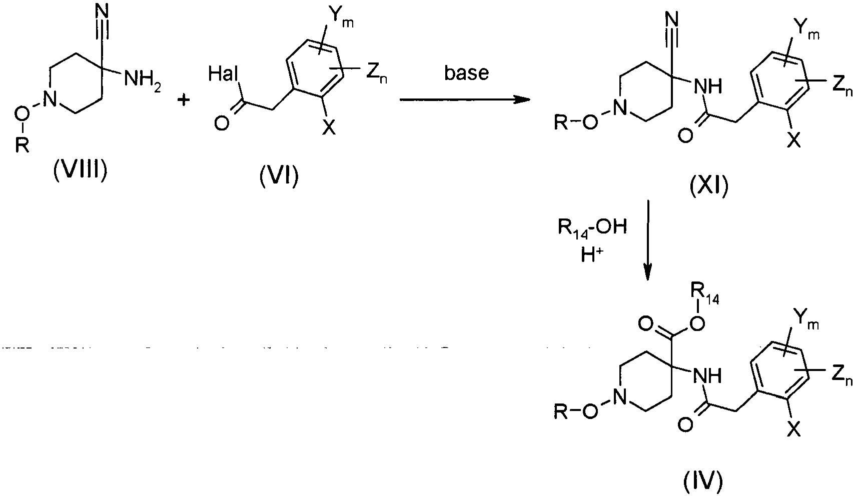

Alternatively, compounds of formula IV, wherein R₁₄ is C₁-C₆alkyl, may be prepared by subjecting derivatives of formula XI to alcoholysis with R₁₄OH, preferably in acidic media (especially sulfuric acid or hydrochloric acid) by known methods described, for example, in US 6774133, US 6555567 and US 6479489. X, Y, Z, m, n and R are as defined above. Compounds of formula XI may be themselves prepared by reacting amino nitriles of formula VIII with phenylacetyl halides of formula VI, preferably in the presence of base in a suitable solvent by known methods described, for example, in US 6774133, US 6555567 and US 6479489. X, Y, Z, m, n and R are as defined above. The base may be inorganic such as an alkali metal carbonate or hydroxide or a metal hydride, or an organic base such as a tertiary amine or metal alkoxide. Examples of suitable inorganic bases include sodium carbonate, sodium or potassium hydroxide, sodium hydride, and suitable organic bases include trialkylamines such as trimethylamine and triethylamine, pyridines or other amine bases such as 1,4-diazobicyclo[2.2.2]octane and 1,8-diazabicyclo[5.4.0]undec-7-ene. Preferred bases include triethylamine and pyridine. Suitable solvents for this reaction are selected to be compatible with the reagents and include ethers such as tetrahydrofuran and 1,2-dimethoxyethane and halogenated solvents such as dichloromethane and chloroform. Certain bases, such as pyridine and triethylamine, may be employed successfully as both base and solvent.

Certain compounds of formula II, III, IV, V, VII, VIII, IX and XI, and salts thereof, are novel and as such form a further aspect of the invention.

The reactants can be reacted in the presence of a base. Examples of suitable bases are alkali metal or alkaline earth metal hydroxides, alkali metal or alkaline earth metal hydrides, alkali metal or alkaline earth metal amides, alkali metal or alkaline earth metal alkoxides, alkali metal or alkaline earth metal acetates, alkali metal or alkaline earth metal carbonates, alkali metal or alkaline earth metal dialkylamides or alkali metal or alkaline earth metal alkylsilylamides, alkylamines, alkylenediamines, free or N-alkylated saturated or unsaturated cycloalkylamines, basic heterocycles, ammonium hydroxides and carbocyclic amines. Examples which may be mentioned are sodium hydroxide, sodium hydride, sodium amide, sodium methoxide, sodium acetate, sodium carbonate, potassium tert-butoxide, potassium hydroxide, potassium carbonate, potassium hydride, lithium diisopropylamide, potassium bis(trimethylsilyl)amide, calcium hydride, triethylamine, diisopropylethylamine, triethylenediamine, cyclohexylamine, N-cyclohexyl-N,N-dimethylamine, N,N-diethylaniline, pyridine, 4-(N,N-dimethylamino)pyridine, quinuclidine, N-methylmorpholine, benzyltrimethylammonium hydroxide and 1,8-diazabicyclo[5.4.0]undec-7-ene (DBU).

The reactants can be reacted with each other as such, i.e. without adding a solvent or diluent. In most cases, however, it is advantageous to add an inert solvent or diluent or a mixture of these. If the reaction is carried out in the presence of a base, bases which are employed in excess, such as triethylamine, pyridine, N-methylmorpholine or N,N-diethylaniline, may also act as solvents or diluents.

The reaction is advantageously carried out in a temperature range from approximately - 80°C to approximately +140°C, preferably from approximately -30°C to approximately +100°C, in many cases in the range between ambient temperature and approximately +80°C.

A compound I can be converted in a manner known per se into another compound I by replacing one or more substituents of the starting compound I in the customary manner by (an)other substituent(s) according to the invention.

Depending on the choice of the reaction conditions and starting materials which are suitable in each case, it is possible, for example, in one reaction step only to replace one substituent by another substituent according to the invention, or a plurality of substituents can be replaced by other substituents according to the invention in the same reaction step. Salts of compounds I can be prepared in a manner known per se. Thus, for example, acid addition salts of compounds I are obtained by treatment with a suitable acid or a suitable ion exchanger reagent and salts with bases are obtained by treatment with a suitable base or with a suitable ion exchanger reagent.

Salts of compounds I can be converted in the customary manner into the free compounds I, acid addition salts, for example, by treatment with a suitable basic compound or with a suitable ion exchanger reagent and salts with bases, for example, by treatment with a suitable acid or with a suitable ion exchanger reagent.

Salts of compounds I can be converted in a manner known per se into other salts of compounds I, acid addition salts, for example, into other acid addition salts, for example by treatment of a salt of inorganic acid such as hydrochloride with a suitable metal salt such as a sodium, barium or silver salt, of an acid, for example with silver acetate, in a suitable solvent in which an inorganic salt which forms, for example silver chloride, is insoluble and thus precipitates from the reaction mixture.

Depending on the procedure or the reaction conditions, the compounds I, which have salt-forming properties can be obtained in free form or in the form of salts.

The compounds I and, where appropriate, the tautomers thereof, in each case in free form or in salt form, can be present in the form of one of the isomers which are possible or as a mixture of these, for example in the form of pure isomers, such as antipodes and/or diastereomers, or as isomer mixtures, such as enantiomer mixtures, for example racemates, diastereomer mixtures or racemate mixtures, depending on the number, absolute and relative configuration of asymmetric carbon atoms which occur in the molecule and/or depending on the configuration of non-aromatic double bonds which occur in the molecule; the invention relates to the pure isomers and also to all isomer mixtures which are possible and is to be understood in each case in this sense hereinabove and hereinbelow, even when stereochemical details are not mentioned specifically in each case.

Diastereomer mixtures or racemate mixtures of compounds I, in free form or in salt form, which can be obtained depending on which starting materials and procedures have been chosen can be separated in a known manner into the pure diasteromers or racemates on the basis of the physicochemical differences of the components, for example by fractional crystallization, distillation and/or chromatography.

Enantiomer mixtures, such as racemates, which can be obtained in a similar manner can be resolved into the optical antipodes by known methods, for example by recrystallization from an optically active solvent, by chromatography on chiral adsorbents, for example high-performance liquid chromatography (HPLC) on acetyl celulose, with the aid of suitable microorganisms, by cleavage with specific, immobilized enzymes, via the formation of inclusion compounds, for example using chiral crown ethers, where only one enantiomer is complexed, or by conversion into diastereomeric salts, for example by reacting a basic end-product racemate with an optically active acid, such as a carboxylic acid, for example camphor, tartaric or malic acid, or sulfonic acid, for example camphorsulfonic acid, and separating the diastereomer mixture which can be obtained in this manner, for example by fractional crystallization based on their differing solubilities, to give the diastereomers, from which the desired enantiomer can be set free by the action of suitable agents, for example basic agents.

Pure diastereomers or enantiomers can be obtained according to the invention not only by separating suitable isomer mixtures, but also by generally known methods of diastereoselective or enantioselective synthesis, for example by carrying out the process according to the invention with starting materials of a suitable stereochemistry.

It is advantageous to isolate or synthesize in each case the biologically more effective isomer, for example enantiomer or diastereomer, or isomer mixture, for example enantiomer mixture or diastereomer mixture, if the individual components have a different biological activity.

The compounds I and, where appropriate, the tautomers thereof, in each case in free form or in salt form, can, if appropriate, also be obtained in the form of hydrates and/or include other solvents, for example those which may have been used for the crystallization of compounds which are present in solid form.

The compounds according to the following Tables 1 to 36 below can be prepared according to the methods described above. The examples which follow are intended to illustrate the invention and show preferred compounds of formula I.

**Table 1: This table discloses the 132 compounds T1.001 to T1.132 of the formula la:**

| | | | | |
|---|---|---|---|---|
| 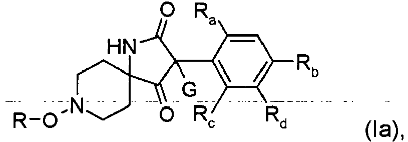 | | | | |
| wherein R is CH₃, G is chlorine and Rₐ, R_{b}, R_{c} and R_{d} are as defined below: | | | | |

| No. | Rₐ | R_{b} | R_{c} | R_{d} |
|---|---|---|---|---|
| T1.001 | Br | H | H | H |
| T1.002 | Cl | H | H | H |
| T1.003 | CH₃ | H | H | H |
| T1.004 | CH₂CH₃ | H | H | H |
| T1.005 | OCH₃ | H | H | H |
| T1.006 | Br | Cl | H | H |
| T1.007 | Cl | Br | H | H |
| T1.008 | Cl | Cl | H | H |
| T1.009 | Cl | CH₃ | H | H |
| T1.010 | CH₃ | Cl | H | H |
| T1.011 | CH₃ | CH₃ | H | H |
| T1.012 | Cl | H | Cl | H |
| T1.013 | Cl | H | CH₃ | H |
| T1.014 | Cl | H | CH₂CH₃ | H |
| T1.015 | Cl | H | OCH₃ | H |
| T1.016 | CH₃ | H | CH₃ | H |
| T1.017 | CH₃ | H | CH₂CH₃ | H |
| T1.018 | CH₃ | H | OCH₃ | H |
| T1.019 | CH₂CH₃ | H | CH₂CH₃ | H |
| T1.020 | CH₂CH₃ | H | OCH₃ | H |
| T1.021 | OCH₃ | H | OCH₃ | H |
| T1.022 | Br | H | H | Cl |
| T1.023 | Br | H | H | CH₃ |
| T1.024 | Br | H | H | 4-Cl-C₆H₄ |
| T1.025 | Cl | H | H | Cl |
| T1.026 | Cl | H | H | CH₃ |
| T1.027 | Cl | H | H | 4-Cl-C₆H₄ |
| T1.028 | CH₃ | H | H | Br |
| T1.029 | CH₃ | H | H | Cl |
| T1.030 | CH₃ | H | H | CH₃ |
| T1.031 | CH₃ | H | H | C₆H₅ |
| T1.032 | CH₃ | H | H | 4-Cl-C₆H₄ |
| T1.033 | CH₂CH₃ | H | H | CH₃ |
| T1.034 | CH₂CH₃ | H | H | 4-Cl-C₆H₄ |
| T1.035 | OCH₃ | H | H | CH₃ |
| T1.036 | OCH₃ | H | H | 4-Cl-C₆H₄ |
| T1.037 | Cl | H | Cl | Br |
| T1.038 | CH₃ | H | CH₃ | Br |
| T1.039 | CH₃ | H | CH₃ | Cl |
| T1.040 | CH₃ | H | CH₃ | 4-Cl-C₆H₄ |
| T1.041 | Br | Cl | H | CH₃ |
| T1.042 | Br | CH₃ | H | CH₃ |
| T1.043 | Cl | Cl | H | Cl |
| T1.044 | Cl | Br | H | CH₃ |
| T1.045 | Cl | Cl | H | CH₃ |
| T1.046 | Cl | CH₃ | H | Cl |
| T1.047 | Cl | CH₃ | H | CH₃ |
| T1.048 | CH₃ | Br | H | CH₃ |
| T1.049 | CH₃ | Cl | H | CH₃ |
| T1.050 | CH₃ | CH₃ | H | CH₃ |
| T1.051 | CH₃ | CH₃ | H | 4-Cl-C₆R₄ |
| T1.052 | Br | Br | CH₃ | H |
| T1.053 | Br | Cl | CH₃ | H |
| T1.054 | Br | CH₃ | Br | H |
| T1.055 | Br | CH₃ | Cl | H |
| T1.056 | Cl | Br | CH₃ | H |
| T1.057 | Cl | Cl | Cl | H |
| T1.058 | Cl | Cl | CH₃ | H |
| T1.059 | Cl | CH₃ | Cl | H |
| T1.060 | Cl | CH₃ | CH₂CH₃ | H |
| T1.061 | Cl | CH₃ | OCH₃ | H |
| T1.062 | Cl | 4-Cl-C₆H₄ | Cl | H |
| T1.063 | Cl | 4-Cl-C₆H₄ | CH₃ | H |
| T1.064 | Cl | 4-Cl-c₆H₄ | CH₂CH₃ | H |
| T1.065 | Cl | 4-Cl-C₆H₄ | OCH₃ | H |
| T1.066 | CH₃ | Br | CH₃ | H |
| T1.067 | CH₃ | Cl | CH₃ | H |
| T1.068 | CH₃ | CH₃ | Br | H |
| T1.069 | CH₃ | CH₃ | Cl | H |
| T1.070 | CH₃ | CH₃ | CH₃ | H |
| T1.071 | CH₃ | CH₃ | CH₂CH₃ | H |
| T1.072 | CH₃ | CH₃ | OCH₃ | H |
| T1.073 | CH₃ | 4-Cl-C₆H₄ | CH₃ | H |
| T1.074 | CH₃ | 4-Cl-C₆H₄ | CH₂CH₃ | H |
| T1.075 | CH₃ | 4-Cl-C₆H₄ | OCH₃ | H |
| T1.076 | CH₂CH₃ | Br | Br | H |
| T1.077 | CH₂CH₃ | Br | Cl | H |
| T1.078 | CH₂CH₃ | Br | CH₃ | H |
| T1.079 | CH₂CH₃ | Br | CH₂CH₃ | H |
| T1.080 | CH₂CH₃ | Br | OCH₃ | H |
| T1.081 | CH₂CH₃ | Cl | Br | H |
| T1.082 | CH₂CH₃ | Cl | Cl | H |
| T1.083 | CH₂CH₃ | Cl | CH₃ | H |
| T1.084 | CH₂CH₃ | Cl | CH₂CH₃ | H |
| T1.085 | CH₂CH₃ | Cl | OCH₃ | H |
| T1.086 | CH₂CH₃ | CH₃ | Br | H |
| T1.087 | CH₂CH₃ | CH₃ | Cl | H |
| T1.088 | CH₂CH₃ | CH₃ | CH₂CH₃ | H |
| T1-089 | CH₂CH₃ | CH₃ | OCH₃ | H |
| T1.090 | CH₂CH₃ | CH₂CH₃ | CH₃ | H |
| T1.091 | CH₂CH₃ | CH₂CH₃ | CH₂CH₃ | H |
| T1.092 | CH₂CH₃ | 4-Cl-C₆H₄ | Br | H |
| T1.093 | CH₂CH₃ | 4-Cl-C₆H₄ | CH₂CH₃ | H |
| T1.094 | CH₂CH₃ | 4-Cl-C₆H₄ | OCH₃ | H |
| T1.095 | OCH₃ | Br | CH₃ | H |
| T1.096 | OCH₃ | Cl | CH₃ | H |
| T1.097 | OCH₃ | CH₃ | Br | H |
| T1.098 | OCH₃ | CH₃ | Cl | H |
| T1.099 | OCH₃ | CH₃ | OCH₃ | H |
| T1.100 | OCH₃ | 4-Cl-C₆H₄ | OCH₃ | H |
| T1.101 | CH₃ | CH₃ | CH₃ | F |
| T1.102 | CH₃ | CH₃ | CH₃ | Cl |
| T1.103 | CH₃ | CH₃ | CH₃ | Br |
| T1.104 | CH₃ | CH₃ | CH₃ | CH₃ |
| T1.105 | CH₃ | CH₃ | CH₃ | 4-Cl-C₆H₄ |
| T1.106 | Cl | CH₃ | CH₃ | CH₃ |
| T1.107 | CH₃ | Cl | CH₃ | CH₃ |
| T1.108 | CH₃ | CH₃ | Cl | CH₃ |
| T1.109 | CH₂CH₃ | CH₃ | CH₃ | CH₃ |
| T1.110 | OCH₃ | CH₃ | CH₃ | CH₃ |
| T1.111 | Cyclo-C3 | CH₃ | CH₃ | CH₃ |
| T1.112 | CH₃ | CH₃ | Cyclo-C3 | H |
| T1.113 | CH₃ | F | H | Br |
| T1.114 | CH₃ | CH₃ | H | Br |
| T1.115 | CH₂CH₃ | CH₃ | H | CH₃ |
| T1.116 | OCH₃ | CH₃ | H | CH₃ |
| T1.117 | Cyclo-C3 | CH₃ | H | CH₃ |
| T1.118 | CH₂CH₃ | Cl | H | CH₃ |
| T1.119 | OCH₃ | Cl | H | CH₃ |
| T1.120 | Cyclo-C3 | Cl | H | CH₃ |
| T1.121 | Cl | H | CH₃ | CH₃ |
| T1.122 | CH₃ | H | CH₃ | CH₃ |
| T1.123 | CH₂CH₃ | H | CH₃ | CH₃ |
| T1.124 | OCH₃ | H | CH₃ | CH₃ |
| T1.125 | Cyclo-C3 | H | CH₃ | CH₃ |
| T1.126 | F | H | Cl | CH₃ |
| T1.T27 | Cl | H | F | CH₃ |
| T1.128 | H | CH₃ | CH₃ | CH₃ |
| T1.129 | Br | CH₃ | CH₃ | CH₃ |
| T1.130 | CH₃ | H | Cl | CH₃ |
| T1.131 | CH₃ | H | Br | CH₃ |
| T1.132 | Br | H | CH₃ | CH₃ |

| | | | | |
|---|---|---|---|---|
| Cyclo-C3 means cyclopropyl. | | | | |

Table 2: This table discloses the 132 compounds T2.001 to T2.132 of the formula la, wherein R is CH₂CH₃, G is chlorine and Rₐ, R_{b}, R_{c} and R_{d} are as defined in Table 1.
Table 3: This table discloses the 132 compounds T3.001 to T3.132 of the formula la, wherein R is n-C₃H₇, G is chlorine and Rₐ, R_{b}, R_{c} and R_{d} are as defined in Table 1.
Table 4: This table discloses the 132 compounds T4.001 to T4.132 of the formula la, wherein R is i-C₃H₇, G is chlorine and Rₐ, R_{b}, R_{c} and R_{d} are as defined in Table 1.
Table 5: This table discloses the 132 compounds T5.001 to T5.132 of the formula la, wherein R is allyl, G is chlorine and Rₐ, R_{b}, R_{c} and R_{d} are as defined in Table 1.
Table 6: This table discloses the 132 compounds T6.001 to T6.132 of the formula la, wherein R is propargyl, G is chlorine and Rₐ, R_{b}, R_{c} and R_{d} are as defined in Table 1.
Table 7: This table discloses the 132 compounds T7.001 to T7.132 of the formula la, wherein R is benzyl, G is chlorine and Rₐ, R_{b}, R_{c} and R_{d} are as defined in Table 1.
Table 8: This table discloses the 132 compounds T8.001 to T8.132 of the formula la, wherein R is hydrogen, G is chlorine and Rₐ, R_{b}, R_{c} and R_{d} are as defined in Table 1.
Table 9: This table discloses the 132 compounds T9.001 to T9.132 of the formula la, wherein R is CH₂OCH₃ G is chlorine and Rₐ, R_{b}, R_{c} and R_{d} are as defined in Table 1.
Table 10: This table discloses the 132 compounds T10.001 to T10.132 of the formula la, wherein R is CH₂CH₂OCH₃, G is chlorine and Rₐ, R_{b}, R_{c} and R_{d} are as defined in Table 1.
Table 11: This table discloses the 132 compounds T11.001 to T11.132 of the formula la, wherein R is CH₂OCH₂CH₃, G is chlorine and Rₐ, R_{b}, R_{c} and R_{d} are as defined in Table 1.
Table 12: This table discloses the 132 compounds T12.001 to T12.132 of the formula la, wherein R is CH₂OC₂H₄OCH₃, G is chlorine and Rₐ, R_{b}, R_{c} and R_{d} are as defined in Table 1.
Table 13: This table discloses the 132 compounds T13.001 to T13.132 of the formula la, wherein R is CH₃, G is fluorine and Rₐ, R_{b}, R_{c} and R_{d} are as defined in Table 1.
Table 14: This table discloses the 132 compounds T14.001 to T14.132 of the formula la, wherein R is CH₂CH₃, G is fluorine and Rₐ, R_{b}, R_{c} and R_{d} are as defined in Table 1.
Table 15: This table discloses the 132 compounds T15.001 to T15.132 of the formula la, wherein R is allyl, G is fluorine and Rₐ, R_{b}, R_{c} and R_{d} are as defined in Table 1.
Table 16: This table discloses the 132 compounds T16.001 to T16.132 of the formula la, wherein R is propargyl, G is fluorine and Rₐ, R_{b}, R_{c} and R_{d} are as defined in Table 1.
Table 17: This table discloses the 132 compounds T17.001 to T17.132 of the formula la, wherein R is benzyl, G is fluorine and Rₐ, R_{b}, R_{c} and R_{d} are as defined in Table 1.
Table 18: This table discloses the 132 compounds T18.001 to T18.132 of the formula la, wherein R is hydrogen, G is fluorine and Rₐ, R_{b}, R_{c} and R_{d} are as defined in Table 1.
Table 19: This table discloses the 132 compounds T19.001 to T19.132 of the formula la, wherein R is CH₂OCH₃, G is fluorine and Rₐ, R_{b}, R_{c} and R_{d} are as defined in Table 1.
Table 20: This table discloses the 132 compounds T20.001 to T20.132 of the formula la, wherein R is CH₂CH₂OCH₃, G is fluorine and Rₐ, R_{b}, R_{c} and R_{d} are as defined in Table 1.
Table 21: This table discloses the 132 compounds T21.001 to T21.132 of the formula la, wherein R is CH₃, G is bromine and Rₐ, R_{b}, R_{c} and R_{d} are as defined in Table 1.
Table 22: This table discloses the 132 compounds T22.001 to T22.132 of the formula la, wherein R is CH₂CH₃, G is bromine and Rₐ, R_{b}, R_{c} and R_{d} are as defined in Table 1.
Table 23: This table discloses the 132 compounds T23.001 to T23.132 of the formula la, wherein R is allyl, G is bromine and Rₐ, R_{b}, R_{c} and R_{d} are as defined in Table 1.
Table 24: This table discloses the 132 compounds T24.001 to T24.132 of the formula la, wherein R is propargyl, G is bromine and Rₐ, R_{b}, R_{c} and R_{d} are as defined in Table 1.
Table 25: This table discloses the 132 compounds T25.001 to T25.132 of the formula la, wherein R is benzyl, G is bromine and Rₐ, R_{b}, R_{c} and R_{d} are as defined in Table 1.
Table 26: This table discloses the 132 compounds T26.001 to T26.132 of the formula la, wherein R is hydrogen, G is bromine and Rₐ, R_{b}, R_{c} and R_{d} are as defined in Table 1.
Table 27: This table discloses the 132 compounds T27.001 to T27.132 of the formula la, wherein R is CH₂OCH₃, G is bromine and Rₐ, R_{b}, R_{c} and R_{d} are as defined in Table 1.
Table 28: This table discloses the 132 compounds T28.001 to T28.132 of the formula la, wherein R is CH₂CH₂OCH₃, G is bromine and Rₐ, R_{b}, R_{c} and R_{d} are as defined in Table 1.
Table 29: This table discloses the 132 compounds T29.001 to T29.132 of the formula la, wherein R is CH₃, G is nitro and Rₐ, R_{b}, R_{c} and R_{d} are as defined in Table 1.
Table 30: This table discloses the 132 compounds T30.001 to T30.132 of the formula la, wherein R is CH₂CH₃, G is nitro and Rₐ, R_{b}, R_{c} and R_{d} are as defined in Table 1.
Table 31: This table discloses the 132 compounds T31.001 to T31.132 of the formula la, wherein R is allyl, G is nitro and Rₐ, R_{b}, R_{c} and R_{d} are as defined in Table 1.
Table 32: This table discloses the 132 compounds T32.001 to T32.132 of the formula la, wherein R is propargyl, G is nitro and Rₐ, R_{b}, R_{c} and R_{d} are as defined in Table 1.
Table 33: This table discloses the 132 compounds T33.001 to T33.132 of the formula la, wherein R is benzyl, G is nitro and Rₐ, R_{b}, R_{c} and R_{d} are as defined in Table 1.
Table 34: This table discloses the 132 compounds T34.001 to T34.132 of the formula la, wherein R is hydrogen, G is nitro and Rₐ, R_{b}, R_{c} and R_{d} are as defined in Table 1.
Table 35: This table discloses the 132 compounds T35.001 to T35.132 of the formula la, wherein R is CH₂OCH₃, G is nitro and Rₐ, R_{b}, R_{c} and R_{d} are as defined in Table 1.
Table 36: This table discloses the 132 compounds T36.001 to T36.132 of the formula la, wherein R is CH₂CH₂OCH₃, G is nitro and Rₐ, R_{b}, R_{c} and R_{d} are as defined in Table 1.

The compounds according to the invention are preventively and/or curatively valuable active ingredients in the field of pest control, even at low rates of application, which have a very favorable biocidal spectrum and are well tolerated by warm-blooded species, fish and plants. The active ingredients according to the invention act against all or individual developmental stages of normally sensitive or resistant pests, such as insects or representatives of the order Acarina. The insecticidal or acaricidal activity of the active ingredients according to the invention can manifest itself directly, i. e. in destruction of the pests, which takes place either immediately or only after some time has elapsed, for example during ecdysis, or indirectly, for example in a reduced oviposition and/or hatching rate, a good activity corresponding to a destruction rate (mortality) of at least 50 to 60%.

The compounds of formula I can be used to combat and control infestations of insect pests such as Lepidoptera, Diptera, Hemiptera, Thysanoptera, Orthoptera, Dictyoptera, Coleoptera, Siphonaptera, Hymenoptera and Isoptera and also other invertebrate pests, for
example, acarine, nematode and mollusc pests. Insects, acarines, nematodes and molluscs are hereinafter collectively referred to as pests. The pests which may be combated and controlled by the use of the invention compounds include those pests associated with agriculture (which term includes the growing of crops for food and fibre products), horticulture and animal husbandry, companion animals, forestry and the storage of products of vegetable origin (such as fruit, grain and timber); those pests associated with the damage of man-made structures and the transmission of diseases of man and animals; and also nuissance pests (such as flies).

Examples of pest species which may be controlled by the compounds of formula I include: *Myzus persicae* (aphid), *Aphis gossypii* (aphid), *Aphis fabae* (aphid), *Lygus* spp. (capsids), *Dysdercus* spp. (capsids), *Nilaparvata lugens* (planthopper), *Nephotettixc incticeps* (leafhopper), *Nezara* spp. (stinkbugs), *Euschistus* spp. (stinkbugs), *Leptocorisa* spp. (stinkbugs), *Frankliniella occidentalis* (thrip), Thrips spp. (thrips), *Leptinotarsa decemlineata* (Colorado potato beetle), *Anthonomus grandis* (boll weevil), *Aonidiella* spp. (scale insects), *Trialeurodes* spp. (white flies), *Bemisia tabaci* (white fly), *Ostrinia nubilalis* (European corn borer), *Spodoptera littoralis* (cotton leafworm), *Heliothis virescens* (tobacco budworm), *Helicoverpa armigera* (cotton bollworm), *Helicoverpa zea* (cotton bollworm), *Sylepta derogata* (cotton leaf roller), *Pieris brassicae* (white butterfly), *Plutella xylostella* (diamond back moth), *Agrotis* spp. (cutworms), *Chilo suppressalis* (rice stem borer), *Locusta migratoria* (locust), *Chortiocetes terminifera* (locust), *Diabrotica* spp. (rootworms), *Panonychus ulmi* (European red mite), *Panonychus citri* (citrus red mite), *Tetranychus urticae* (two-spotted spider mite), *Tetranychus cinnabarinus* (carmine spider mite), *Phyllocoptruta oleivora* (citrus rust mite), *Polyphagotarsonemus latus* (broad mite), *Brevipalpus* spp. (flat *mites), Boophilus microplus* (cattle tick), *Dermacentor variabilis* (American dog tick), *Ctenocephalides felis* (cat flea), *Liriomyza* spp. (leafminer), *Musca domestica* (housefly), *Aedes aegypti* (mosquito), *Anopheles* spp. (mosquitoes), *Culex* spp. (mosquitoes), *Lucillia* spp. (blowflies), *Blattella germanica* (cockroach), *Periplaneta americana* (cockroach), *Blatta orientalis* (cockroach), termites of the Mastotermitidae (for example *Mastotermes* spp.), the Kalotermitidae (for example *Neotermes* spp.), the Rhinotermitidae (for example *Coptotermes formosanus, Reticulitermes flavipes, R. speratu, R. virginicus, R. hesperus,* and *R. santonensis)* and the Termitidae (for example *Globitermes sulphureus), Solenopsis geminata* (fire ant), *Monomorium pharaonis* (pharaoh's ant), *Damalinia* spp. and *Linognathus* spp. (biting and sucking lice), *Meloidogyne* spp. (root knot nematodes), *Globodera* spp. and *Heterodera* spp. (cyst nematodes), *Pratylenchus* spp. (lesion nematodes), *Rhodopholus* spp. (banana burrowing nematodes), *Tylenchulus* spp.(citrus nematodes), *Haemonchus contortus* (barber pole worm), *Caenorhabditis elegans*_(vinegar eelworm), *Trichostrongylus* spp. (gastro intestinal nematodes) and *Deroceras reticulatum* (slug).

Further examples of the above mentioned pests are:
from the order *Acarina,* for example,
   Acarus siro, Aceria sheldoni, Aculus schlechtendali, Amblyomma spp., Argas spp., Boophilus spp., Brevipalpus spp., Bryobia praetiosa, Calipitrimerus spp., Chorioptes spp., Dermanyssus gallinae, Eotetranychus carpini, Eriophyes spp., Hyalomma spp., Ixodes spp., Olygonychus pratensis, Ornithodoros spp., Panonychus spp., Phyllocoptruta oleivora, Polyphagotarsonemus latus, Psoroptes spp., Rhipicephalus spp., Rhizoglyphus spp., Sarcoptes spp., Tarsonemus spp. and Tetranychus spp.;
from the order *Anoplura,* for example,
   Haematopinus spp., Linognathus spp., Pediculus spp., Pemphigus spp. and Phylloxera spp.;
from the order *Coleoptera,* for example,
   Agriotes spp., Anthonomus spp., Atomaria linearis, Chaetocnema tibialis, Cosmopolites spp., Curculio spp., Dermestes spp., Diabrotica spp., Epilachna spp., Eremnus spp., Leptinotarsa decemLineata, Lissorhoptrus spp., Melolontha spp., Orycaephilus spp., Otiorhynchus spp., Phlyctinus spp., Popillia spp., Psylliodes spp., Rhizopertha spp., Scarabeidae, Sitophilus spp., Sitotroga spp., Tenebrio spp., Tribolium spp. and Trogoderma spp.;
from the order *Diptera,* for example,
   Aedes spp., Antherigona soccata, Bibio hortulanus, Calliphora erythrocephala, Ceratitis spp., Chrysomyia spp., Culex spp., Cuterebra spp., Dacus spp., Drosophila melanogaster, Fannia spp., Gastrophilus spp., Glossina spp., Hypoderma spp., Hyppobosca spp., Liriomyza spp., Lucilia spp., Melanagromyza spp., Musca spp., Oestrus spp., Orseolia spp., Oscinella frit, Pegomyia hyoscyami, Phorbia spp., Rhagoletis pomonella, Sciara spp., Stomoxys spp., Tabanus spp., Tannia spp. and Tipula spp.;
from the order *Heteroptera,* for example,
   Cimex spp., Distantiella theobroma, Dysdercus spp., Euchistus spp., Eurygaster spp., Leptocorisa spp., Nezara spp., Piesma spp., Rhodnius spp., Sahlbergella singularis, Scotinophara spp. and Triatoma spp.;
from the order *Homoptera,* for example,
   Aleurothrixus floccosus, Aleyrodes brassicae, Aonidiella spp., Aphididae, Aphis spp., Aspidiotus spp., Bemisia tabaci, Ceroplaster spp., Chrysomphalus aonidium, Chrysomphalus dictyospermi, Coccus hesperidum, Empoasca spp., Eriosoma larigerum, Erythroneura spp., Gascardia spp., Laodelphax spp., Lecanium corni, Lepidosaphes spp., Macrosiphus spp., Myzus spp., Nephotettix spp., Nilaparvata spp., Parlatoria spp., Pemphigus spp., Planococcus spp., Pseudaulacaspis spp., Pseudococcus spp., Psylla spp., Pulvinaria aethiopica, Quadraspidiotus spp., Rhopalosiphum spp., Saissetia spp., Scaphoideus spp., Schizaphis spp., Sitobion spp., Trialeurodes vaporariorum, Trioza erytreae and Unaspis citri;
from the order *Hymenoptera,* for example,
   Acromyrmex, Atta spp., Cephus spp., Diprion spp., Diprionidae, Gilpinia polytoma, Hoplocampa spp., Lasius spp., Monomorium pharaonis, Neodiprion spp., Solenopsis spp. and Vespa spp.;
from the order *Isoptera,* for example,
   Reticulitermes spp.;
from the order *Lepidoptera,* for example,
   Acleris spp., Adoxophyes spp., Aegeria spp., Agrotis spp., Alabama argillaceae, Amylois spp., Anticarsia gemmatalis, Archips spp., Argyrotaenia spp., Autographa spp., Busseola fusca, Cadra cautella, Carposina nipponensis, Chilo spp., Choristoneura spp., Clysia ambiguella, Cnaphalocrocis spp., Cnephasia spp., Cochylis spp., Coleophora spp., Crocidolomia binotalis, Cryptophlebia leucotreta, Cydia spp., Diatraea spp., Diparopsis castanea, Earias spp., Ephestia spp., Eucosma spp., Eupoecilia ambiguella, Euproctis spp., Euxoa spp., Grapholita spp., Hedya nubiferana, Heliothis spp., Hellula undalis, Hyphantria cunea, Keiferia lycopersicella, Leucoptera scitella, Lithocollethis spp., Lobesia botrana, Lymantria spp., Lyonetia spp., Malacosoma spp., Mamestra brassicae, Manduca sexta, Operophtera spp., Ostrinia nubilalis, Pammene spp., Pandemis spp., Panolis flammea, Pectinophora gossypiela, Phthorimaea operculella, Pieris rapae, Pieris spp., Plutella xylostella, Prays spp., Scirpophaga spp., Sesamia spp., Sparganothis spp., Spodoptera spp., Synanthedon spp., Thaumetopoea spp., Tortrix spp., Trichoplusia ni and Yponomeuta spp.;
from the order *Mallophaga,* for example,
   Damalinea spp. and Trichodectes spp.;
from the order *Orthoptera,* for example,
   Blatta spp., Blattella spp., Gryllotalpa spp., Leucophaea maderae, Locusta spp., Periplaneta spp. and Schistocerca spp.;
from the order *Psocoptera,* for example,
   Liposcelis spp.;
from the order *Siphonaptera,* for example,
   Ceratophyllus spp., Ctenocephalides spp. and Xenopsylla cheopis;
from the order *Thysanoptera,* for example,
   Frankliniella spp., Hercinothrips spp., Scirtothrips aurantii, Taeniothrips spp., Thrips palmi and Thrips tabaci; and
from the order *Thysanura,* for example,
   Lepisma saccharina.

The active ingredients according to the invention can be used for controlling, i. e. containing or destroying, pests of the abovementioned type which occur in particular on plants, especially on useful plants and ornamentals in agriculture, in horticulture and in forests, or on organs, such as fruits, flowers, foliage, stalks, tubers or roots, of such plants, and in some cases even plant organs which are formed at a later point in time remain protected against these pests.

Suitable target crops are, in particular, cereals, such as wheat, barley, rye, oats, rice, maize or sorghum; beet, such as sugar or fodder beet; fruit, for example pomaceous fruit, stone fruit or soft fruit, such as apples, pears, plums, peaches, almonds, cherries or berries, for example strawberries, raspberries or blackberries; leguminous crops, such as beans, lentils, peas or soya; oil crops, such as oilseed rape, mustard, poppies, olives, sunflowers, coconut, castor, cocoa or ground nuts; cucurbits, such as pumpkins, cucumbers or melons; fibre plants, such as cotton, flax, hemp or jute; citrus fruit, such as oranges, lemons, grapefruit or tangerines; vegetables, such as spinach, lettuce, asparagus, cabbages, carrots, onions, tomatoes, potatoes or bell peppers; Lauraceae, such as avocado, Cinnamonium or camphor; and also tobacco, nuts, coffee, eggplants, sugarcane, tea, pepper, grapevines, hops, the plantain family, latex plants and ornamentals.

The term "crops" is to be understood as including also crops that have been rendered tolerant to herbicides like bromoxynil or classes of herbicides (such as, for example, HPPD inhibitors, ALS inhibitors, for example primisulfuron, prosulfuron and trifloxysulfuron, EPSPS (5-enol-pyrovyl-shikimate-3-phosphate-synthase) inhibitors, GS (glutamine synthetase) inhibitors) as a result of conventional methods of breeding or genetic engineering. An example of a crop that has been rendered tolerant to imidazolinones, e.g. imazamox, by conventional methods of breeding (mutagenesis) is Clearfield® summer rape (Canola). Examples of crops that have been rendered tolerant to herbicides or classes of herbicides by genetic engineering methods include glyphosate- and glufosinate-resistant maize varieties commercially available under the trade names RoundupReady® and LibertyLink®.

The term "crops" is also to be understood as including also crop plants which have been so transformed by the use of recombinant DNA techniques that they are capable of synthesising one or more selectively acting toxins, such as are known, for example, from toxin-producing bacteria, especially those of the genus Bacillus.

Toxins that can be expressed by such transgenic plants include, for example, insecticidal proteins, for example insecticidal proteins from Bacillus cereus or Bacillus popliae; or insecticidal proteins from Bacillus thuringiensis, such as δ-endotoxins, e.g. CryIA(b), CryIA(c), CryIF, CryIF(a2), CryIIA(b), CryIIIA, CryIIIB(b1) or Cry9c, or vegetative insecticidal proteins (VIP), e.g. VIP1, VIP2, VIP3 or VIP3A; or insecticidal proteins of bacteria colonising nematodes, for example Photorhabdus spp. or Xenorhabdus spp., such as Photorhabdus luminescens, Xenorhabdus nematophilus; toxins produced by animals, such as scorpion toxins, arachnid toxins, wasp toxins and other insect-specific neurotoxins; toxins produced by fungi, such as Streptomycetes toxins, plant lectins, such as pea lectins, barley lectins or snowdrop lectins; agglutinins; proteinase inhibitors, such as trypsine inhibitors, serine protease inhibitors, patatin, cystatin, papain inhibitors; ribosome-inactivating proteins (RIP), such as ricin, maize-RIP, abrin, luffin, saporin or bryodin; steroid metabolism enzymes, such as 3-hydroxysteroidoxidase, ecdysteroid-UDP-glycosyl-transferase, cholesterol oxidases, ecdysone inhibitors, HMG-COA-reductase, ion channel blockers, such as blockers of sodium or calcium channels, juvenile hormone esterase, diuretic hormone receptors, stilbene synthase, bibenzyl synthase, chitinases and glucanases.

In the context of the present invention there are to be understood by δ-endotoxins, for example CryIA(b),CryIA(c), CryIF, CryIF(a2), CryIIA(b), CryIIIA, CryIIIB(b1) or Cry9c, or vegetative insecticidal proteins (VIP), for example VIP1, VIP2, VIP3 or VIP3A, expressly also hybrid toxins, truncated toxins and modified toxins. Hybrid toxins are produced recombinantly by a new combination of different domains of those proteins (see, for example, WO 02/15701). Truncated toxins, for example a truncated CryIA(b), are known. In the case of modified toxins, one or more amino acids of the naturally occurring toxin are replaced. In such amino acid replacements, preferably non-naturally present protease recognition sequences are inserted into the toxin, such as, for example, in the case of CryIIIA055, a cathepsin-D-recognition sequence is inserted into a CryIIIA toxin (see WO 03/018810).

Examples of such toxins or transgenic plants capable of synthesising such toxins are disclosed, for example, in EP-A-0 374 753, WO 93/07278, WO 95/34656, EP-A-0 427 529, EP-A-451 878 and WO 03/052073.

The processes for the preparation of such transgenic plants are generally known to the person skilled in the art and are described, for example, in the publications mentioned above. CryI-type deoxyribonucleic acids and their preparation are known, for example, from WO 95/34656, EP-A-0 367 474, EP-A-0 401 979 and WO 90/13651.

The toxin contained in the transgenic plants imparts to the plants tolerance to harmful insects. Such insects can occur in any taxonomic group of insects, but are especially commonly found in the beetles (Coleoptera), two-winged insects (Diptera) and butterflies (Lepidoptera).

Transgenic plants containing one or more genes that code for an insecticidal resistance and express one or more toxins are known and some of them are commercially available. Examples of such plants are: YieldGard® (maize variety that expresses a CryIA(b) toxin); YieldGard Rootworm® (maize variety that expresses a CryIIIB(b1) toxin); YieldGard Plus® (maize variety that expresses a CryIA(b) and a CryIIIB(b1) toxin); Starlink® (maize variety that expresses a Cry9(c) toxin); Herculex I® (maize variety that expresses a CryIF(a2) toxin and the enzyme phosphinothricine N-acetyltransferase (PAT) to achieve tolerance to the herbicide glufosinate ammonium); NuCOTN 33B® (cotton variety that expresses a CryIA(c) toxin); Bollgard I® (cotton variety that expresses a CryIA(c)toxin); Bollgard II® (cotton variety that expresses a CryIA(c)and a CryIIA(b) toxin); VIPCOT® (cotton variety that expresses a VIP toxin); NewLeaf® (potato variety that expresses a CryIIIA toxin); Nature-Gard® Agrisure® GT Advantage (GA21 glyphosate-tolerant trait), Agrisure® CB Advantage (Bt11 corn borer (CB) trait) and Protecta®.

Further examples of such transgenic crops are:
1. Bt11 Maize from Syngenta Seeds SAS, Chemin de l'Hobit 27, F-31 790 St. Sauveur, France, registration number C/FR/96/05/10. Genetically modified *Zea mays* which has been rendered resistant to attack by the European corn borer *(Ostrinia nubilalis* and *Sesamia nonagrioides)* by transgenic expression of a truncated CryIA(b) toxin. Bt11 maize also transgenically expresses the enzyme PAT to achieve tolerance to the herbicide glufosinate ammonium.
2. Bt176 Maize from Syngenta Seeds SAS, Chemin de l'Hobit 27, F-31 790 St. Sauveur, France, registration number C/FR/96/05/10. Genetically modified *Zea mays* which has been rendered resistant to attack by the European corn borer (*Ostrinia nubilalis* and *Sesamia nonagrioides)* by transgenic expression of a CryIA(b) toxin. Bt176 maize also transgenically expresses the enzyme PAT to achieve tolerance to the herbicide glufosinate ammonium.
3. MIR604 Maize from Syngenta Seeds SAS, Chemin de l'Hobit 27, F-31 790 St. Sauveur, France, registration number C/FR/96/05/10. Maize which has been rendered insect-resistant by transgenic expression of a modified CryIIIA toxin. This toxin is Cry3A055 modified by insertion of a cathepsin-D-protease recognition sequence. The preparation of such transgenic maize plants is described in WO 03/018810.
4. MON 863 Maize from Monsanto Europe S.A. 270-272 Avenue de Tervuren, B-1150 Brussels, Belgium, registration number C/DE/02/9. MON 863 expresses a CryIIIB(b1) toxin and has resistance to certain Coleoptera insects.
5. IPC 531 Cotton from Monsanto Europe S.A. 270-272 Avenue de Tervuren, B-1150 Brussels, Belgium, registration number C/ES/96/02.
6. 1507 Maize from Pioneer Overseas Corporation, Avenue Tedesco, 7 B-1160 Brussels, Belgium, registration number C/NL/00/10. Genetically modified maize for the expression of the protein Cry1F for achieving resistance to certain Lepidoptera insects and of the PAT protein for achieving tolerance to the herbicide glufosinate ammonium.
7. NK603 x MON 810 Maize from Monsanto Europe S.A. 270-272 Avenue de Tervuren, B-1150 Brussels, Belgium, registration number C/GB/02/M3/03. Consists of conventionally bred hybrid maize varieties by crossing the genetically modified varieties NK603 and MON 810. NK603 x MON 810 Maize transgenically expresses the protein CP4 EPSPS, obtained from *Agrobacterium sp.* strain CP4, which imparts tolerance to the herbicide Roundup® (contains glyphosate), and also a CryIA(b) toxin obtained from *Bacillus thuringiensis subsp. kurstaki* which brings about tolerance to certain Lepidoptera, include the European corn borer.

Transgenic crops of insect-resistant plants are also described in BATS (Zentrum für Biosicherheit und Nachhaltigkeit, Zentrum BATS, Clarastrasse 13, 4058 Basel, Switzerland) Report 2003.

The term "crops" is to be understood as including also crop plants which have been so transformed by the use of recombinant DNA techniques that they are capable of synthesising antipathogenic substances having a selective action, such as, for example, the so-called "pathogenesis-related proteins" (PRPs, see e.g. EP-A-0 392 225). Examples of such antipathogenic substances and transgenic plants capable of synthesising such antipathogenic substances are known, for example, from EP-A-0 392 225, WO 95/33818, and EP-A-0 353 191. The methods of producing such transgenic plants are generally known to the person skilled in the art and are described, for example, in the publications mentioned above.

Antipathogenic substances which can be expressed by such transgenic plants include, for example, ion channel blockers, such as blockers for sodium and calcium channels, for example the viral KP1, KP4 or KP6 toxins; stilbene synthases; bibenzyl synthases; chitinases; glucanases; the so-called "pathogenesis-related proteins" (PRPs; see e.g. EP-A-0 392 225); antipathogenic substances produced by microorganisms, for example peptide antibiotics or heterocyclic antibiotics (see e.g. WO 95/33818) or protein or polypeptide factors involved in plant pathogen defence (so-called "plant disease resistance genes", as described in WO 03/000906).

Further areas of use of the compounds and compositions according to the invention are the protection of stored goods and storerooms and the protection of raw materials, such as wood, textiles, floor coverings or buildings, and also in the hygiene sector, especially the protection of humans, domestic animals and productive livestock against pests of the mentioned type.

In the hygiene sector, the compounds and compositions according to the invention are active against ectoparasites such as hard ticks, soft ticks, mange mites, harvest mites, flies (biting and licking), parasitic fly larvae, lice, hair lice, bird lice and fleas.

Examples of such parasites are:
Of the order Anoplurida: Haematopinus spp., Linognathus spp., Pediculus spp. and Phtirus spp., Solenopotes spp..
Of the order Mallophagida: Trimenopon spp., Menopon spp., Trinoton spp., Bovicola spp., Werneckiella spp., Lepikentron spp., Damalina spp., Trichodectes spp. and Felicola spp..
Of the order Diptera and the suborders Nematocerina and Brachycerina, for example Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp. and Melophagus spp..
Of the order Siphonapterida, for example Pulex spp., Ctenocephalides spp., Xenopsylla spp., Ceratophyllus spp..
Of the order Heteropterida, for example Cimex spp., Triatoma spp., Rhodnius spp., Panstrongylus spp..
Of the order Blattarida, for example Blatta orientalis, Periplaneta americana, Blattelagermanica and Supella spp..
Of the subclass Acaria (Acarida) and the orders Meta- and Meso-stigmata, for example Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp. and Varroa spp..
Of the orders Actinedida (Prostigmata) and Acaridida (Astigmata), for example Acarapis spp., Cheyletiella spp., Ornithocheyletia spp., Myobia spp., Psorergatesspp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp. and Laminosioptes spp..

The compounds and compositions according to the invention are also suitable for protecting against insect infestation in the case of materials such as wood, textiles, plastics, adhesives, glues, paints, paper and card, leather, floor coverings and buildings.

The compositions according to the invention can be used, for example, against the following pests: beetles such as Hylotrupes bajulus, Chlorophorus pilosis, Anobium punctatum, Xestobium rufovillosum, Ptilinuspecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthesrugicollis, Xyleborus spec.,Tryptodendron spec., Apate monachus, Bostrychus capucins, Heterobostrychus brunneus, Sinoxylon spec. and Dinoderus minutus, and also hymenopterans such as Sirex juvencus, Urocerus gigas, Urocerus gigas taignus and Urocerus augur, and termites such as Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis and Coptotermes formosanus, and bristletails such as Lepisma saccharina.

The invention therefore provides a method of combating and controlling insects, acarines, nematodes or molluscs which comprises applying an insecticidally, acaricidally, nematicidally or molluscicidally effective amount of a compound of formula I, or a composition containing a compound of formula I, to a pest, a locus of pest, or to a plant susceptible to attack by a pest, The compounds of formula I are preferably used against insects or acarines.

The term "plant" as used herein includes seedlings, bushes and trees.

The invention therefore also relates to pesticidal compositions such as emulsifiable concentrates, suspension concentrates, directly sprayable or dilutable solutions, spreadable pastes, dilute emulsions, soluble powders, dispersible powders, wettable powders, dusts, granules or encapsulations in polymeric substances, which comprise - at least - one of the active ingredients according to the invention and which are to be selected to suit the intended aims and the prevailing circumstances.

In these compositions, the active ingredient is employed in pure form, a solid active ingredient for example in a specific particle size, or, preferably, together with - at least - one of the auxiliaries conventionally used in the art of formulation, such as extenders, for example solvents or solid carriers, or such as surface-active compounds (surfactants).

Examples of suitable solvents are: unhydrogenated or partially hydrogenated aromatic hydrocarbons, preferably the fractions C8 to C12 of alkylbenzenes, such as xylene mixtures, alkylated naphthalenes or tetrahydronaphthalene, aliphatic or cycloaliphatic hydrocarbons, such as paraffins or cyclohexane, alcohols such as ethanol, propanol or butanol, glycols and their ethers and esters such as propylene glycol, dipropylene glycol ether, ethylene glycol or ethylene glycol monomethyl ether or ethylene glycol monoethyl ether, ketones, such as cyclohexanone, isophorone or diacetone alcohol, strongly polar solvents, such as N-methylpyrrolid-2-one, dimethyl sulfoxide or N,N-dimethylformamide, water, unepoxidized or epoxidized vegetable oils, such as unexpodized or epoxidized rapeseed, castor, coconut or soya oil, and silicone oils.

Solid carriers which are used for example for dusts and dispersible powders are, as a rule, ground natural minerals such as calcite, talc, kaolin, montmorillonite or attapulgite. To improve the physical properties, it is also possible to add highly disperse silicas or highly disperse absorbtive polymers. Suitable particulate adsorptive carriers for granules are porous types, such as pumice, brick grit, sepiolite or bentonite, and suitable non-sorptive carrier materials are calcite or sand. In addition, a large number of granulated materials of inorganic or organic nature can be used, in particular dolomite or comminuted plant residues.

Suitable surface-active compounds are, depending on the type of the active ingredient to be formulated, non-ionic, cationic and/or anionic surfactants or surfactant mixtures which have good emulsifying, dispersing and wetting properties. The surfactants mentioned below are only to be considered as examples; a large number of further surfactants which are conventionally used in the art of formulation and suitable according to the invention are described in the relevant literature.

Suitable non-ionic surfactants are, especially, polyglycol ether derivatives of aliphatic or cycloaliphatic alcohols, of saturated or unsaturated fatty acids or of alkyl phenols which may contain approximately 3 to approximately 30 glycol ether groups and approximately 8 to approximately 20 carbon atoms in the (cyclo)aliphatic hydrocarbon radical or approximately 6 to approximately 18 carbon atoms in the alkyl moiety of the alkyl phenols. Also suitable are water-soluble polyethylene oxide adducts with polypropylene glycol, ethylene diaminopo¬lypropylene glycol or alkyl polypropylene glycol having 1 to approximately 10 carbon atoms in the alkyl chain and approximately 20 to approximately 250 ethylene glycol ether groups and approximately 10 to approximately 100 propylene glycol ether groups. Normally, the abovementioned compounds contain 1 to approximately 5 ethylene glycol units per propy¬lene glycol unit. Examples which may be mentioned are nonylphenoxypolyethoxyethanol, castor oil polyglycol ether, polypropylene glycol/polyethylene oxide adducts, tributylpheno¬xypolyethoxyethanol, polyethylene glycol or octylphenoxypolyethoxyethanol. Also suitable are fatty acid esters of polyoxyethylene sorbitan, such as polyoxyethylene sorbitan trioleate.

The cationic surfactants are, especially, quarternary ammonium salts which generally have at least one alkyl radical of approximately 8 to approximately 22 C atoms as substituents and as further substituents (unhalogenated or halogenated) lower alkyl or hydroxyalkyl or benzyl radicals. The salts are preferably in the form of halides, methylsulfates or ethylsulfates. Examples are stearyltrimethylammonium chloride and benzylbis(2-chloroethyl)ethyl¬ammonium bromide.

Examples of suitable anionic surfactants are water-soluble soaps or water-soluble synthetic surface-active compounds. Examples of suitable soaps are the alkali, alkaline earth or (unsubstituted or substituted) ammonium salts of fatty acids having approximately 10 to approximately 22 C atoms, such as the sodium or potassium salts of oleic or stearic acid, or of natural fatty acid mixtures which are obtainable for example from coconut or tall oil; mention must also be made of the fatty acid methyl taurates. However, synthetic surfactants are used more frequently, in particular fatty sulfonates, fatty sulfates, sulfonated benzimidazole derivatives or alkylaryl sulfonates. As a rule, the fatty sulfonates and fatty sulfates are present as alkali, alkaline earth or (substituted or unsubstituted) ammonium salts and they generally have an alkyl radical of approximately 8 to approximately 22 C atoms, alkyl also to be understood as including the alkyl moiety of acyl radicals; examples which may be mentioned are the sodium or calcium salts of lignosulfonic acid, of the dodecylsulfuric ester or of a fatty alcohol sulfate mixture prepared from natural fatty acids. This group also includes the salts of the sulfuric esters and sulfonic acids of fatty alcohol/ethylene oxide adducts. The sulfonated benzimidazole derivatives preferably contain 2 sulfonyl groups and a fatty acid radical of approximately 8 to approximately 22 C atoms. Examples of alkylarylsulfonates are the sodium, calcium or triethanolammonium salts of decylbenzenesulfonic acid, of dibutyl¬naphthalenesulfonic acid or of a naphthalenesulfonic acid/formaldehyde condensate. Also possible are, furthermore, suitable phosphates, such as salts of the phosphoric ester of a p-nonylphenol/(4-14)ethylene oxide adduct, or phospholipids. Further suitable phosphates are tris-esters of phosphoric acid with aliphatic or aromatic alcohols and/or bis-esters of alkyl phosphonic acids with aliphatic or aromatic alcohols, which are a high performance oil-type adjuvant. These tris-esters have been described, for example, in WO0147356, WO0056146, EP-A-0579052 or EP-A-1018299 or are commercially available under their chemical name. Preferred tris-esters of phosphoric acid for use in the new compositions are tris-(2-ethylhexyl) phosphate, tris-n-octyl phosphate and tris-butoxyethyl phosphate, where tris-(2-ethylhexyl) phosphate is most preferred. Suitable bis-ester of alkyl phosphonic acids are bis-(2-ethylhexyl)-(2-ethylhexyl)-phosphonate, bis-(2-ethylhexyl)-(n-octyl)-phosphonate, dibutyl-butyl phosphonate and bis(2-ethylhexyl)-tripropylene-phosphonate, where bis-(2-ethylhexyl)-(n-octyl)-phosphonate is particularly preferred.

The compositions according to the invention can preferably additionally include an additive comprising an oil of vegetable or animal origin, a mineral oil, alkyl esters of such oils or mixtures of such oils and oil derivatives. The amount of oil additive used in the composition according to the invention is generally from 0.01 to 10 %, based on the spray mixture. For example, the oil additive can be added to the spray tank in the desired concentration after the spray mixture has been prepared. Preferred oil additives comprise mineral oils or an oil of vegetable origin, for example rapeseed oil such as ADIGOR® and MERO®" olive oil or sunflower oil, emulsified vegetable oil, such as AMIGO® (Rhône-Poulenc Canada Inc.), alkyl esters of oils of vegetable origin, for example the methyl derivatives, or an oil of animal origin, such as fish oil or beef tallow. A preferred additive contains, for example, as active components essentially 80 % by weight alkyl esters of fish oils and 15 % by weight methylated rapeseed oil, and also 5 % by weight of customary emulsifiers and pH modifiers. Especially preferred oil additives comprise alkyl esters of C₈-C₂₂ fatty acids, especially the methyl derivatives of C₁₂-C₁₈ fatty acids, for example the methyl esters of lauric acid, palmitic acid and oleic acid, being important. Those esters are known as methyl laurate (CAS-111-82-0), methyl palmitate (CAS-112-39-0) and methyl oleate (CAS-112-62-9). A preferred fatty acid methyl ester derivative is Emery® 2230 and 2231 (Cognis GmbH). Those and other oil derivatives are also known from the Compendium of Herbicide Adjuvants, 5th Edition, Southern Illinois University, 2000. Also, alkoxylated fatty acids can be used as additives in the inventive compositions as well as polymethylsiloxane based additives, which have been described in WO08/037373.

The application and action of the oil additives can be further improved by combining them with surface-active substances, such as non-ionic, anionic or cationic surfactants. Examples of suitable anionic, non-ionic and cationic surfactants are listed on pages 7 and 8 of WO 97/34485. Preferred surface-active substances are anionic surfactants of the dodecylbenzylsulfonate type, especially the calcium salts thereof, and also non-ionic surfactants of the fatty alcohol ethoxylate type. Special preference is given to ethoxylated C₁₂-C₂₂ fatty alcohols having a degree of ethoxylation of from 5 to 40. Examples of commercially available surfactants are the Genapol types (Clariant AG). Also preferred are silicone surfactants, especially polyalkyl-oxide-modified heptamethyltrisiloxanes, which are commercially available e.g. as Silwet L-77®, and also perfluorinated surfactants. The concentration of surface-active substances in relation to the total additive is generally from 1 to 30 % by weight. Examples of oil additives that consist of mixtures of oils or mineral oils or derivatives thereof with surfactants are Edenor ME SU®, Turbocharge® (Syngenta AG, CH) and Actipron® (BP Oil UK Limited, GB).

The said surface-active substances may also be used in the formulations alone, that is to say without oil additives.

Furthermore, the addition of an organic solvent to the oil additive/surfactant mixture can contribute to a further enhancement of action. Suitable solvents are, for example, Solvesso® (ESSO) and Aromatic Solvent® (Exxon Corporation).The concentration of such solvents can be from 10 to 80 % by weight of the total weight. Such oil additives, which may be in admixture with solvents, are described, for example, in US-A-4 834 908. A commercially available oil additive disclosed therein is known by the name MERGE® (BASF Corporation). A further oil additive that is preferred according to the invention is SCORE® (Syngenta Crop Protection Canada.)

In addition to the oil additives listed above, in order to enhance the activity of the compositions according to the invention it is also possible for formulations of alkylpyrrolidones, (e.g. Agrimax®) to be added to the spray mixture. Formulations of synthetic latices, such as, for example, polyacrylamide, polyvinyl compounds or poly-1-p-menthene (e.g. Bond®, Courier® or Emerald®) can also be used. Solutions that contain propionic acid, for example Eurogkem Pen-e-trate®, can also be mixed into the spray mixture as activity-enhancing agents.

As a rule, the compositions comprise 0.1 to 99%, especially 0.1 to 95%, of active ingredient and 1 to 99.9%, especially 5 to 99.9%, of at least one solid or liquid adjuvant, it being possible as a rule for 0 to 25%, especially 0.1 to 20%, of the composition to be surfactants(% in each case meaning percent by weight). Whereas concentrated compositions tend to be preferred for commercial goods, the end consumer as a rule uses dilute compositions which have substantially lower concentrations of active ingredient. Preferred compositions are composed in particular as follows (% = percent by weight):

| Emulsifiable concentrates: | |
|---|---|
| active ingredient: | 1 to 95%, preferably 5 to 50%, more preferably 5 to 20% |
| surfactant: | 1 to 30%, preferably 10 to 20 % |
| solvent: | 5 to 98%, preferably 70 to 85% |
| | |

| Dusts: | |
|---|---|
| active ingredient: | 0.1 to 10%, preferably 0.1 to 1% |
| solid carrier: | 99.9 to 90%, preferably 99.9 to 99% |
| | |

| Suspension concentrates: | |
|---|---|
| active ingredient: | 5 to 75%, preferably 10 to 50%, more preferably 10 to 40% |
| water: | 94 to 24%, preferably 88 to 30% |
| surfactant: | 1 to 40%, preferably 2 to 30% |
| | |

| Oil-based suspension concentrates: | |
|---|---|
| active ingredient: | 2 to 75%, preferably 5 to 50% |
| oil: | 94 to 24%, preferably 88 to 30% |
| surfactant: | 1 to 40%, preferably 2 to 30% |
| | |

| Wettable powders: | |
|---|---|
| active ingredient: | 0.5 to 90%, preferably 1 to 80%, more preferably 25 to 75% |
| surfactant: | 0.5 to 20%, preferably 1 to 15% |
| solid carrier: | 5 to 99%, preferably 15 to 98% |
| | |

| Granulates: | |
|---|---|
| active ingredient: | 0.5 to 30%, preferably 3 to 15%, more preferably 3 to 15% |
| solid carrier: | 99.5 to 70%, preferably 97 to 85% |

Preferably, the term "active ingredient" refers to one of the compounds selected from Tables 1 to 36 shown above. It also refers to mixtures of the compound of formula I, in particular a compound selected from said Tables 1 to 36, with other insecticides, fungicides, herbicides, safeners, adjuvants and the like, which mixtures are specifically disclosed below.

The compositions can also comprise further solid or liquid auxiliaries, such as stabilizers, for example unepoxidized or epoxidized vegetable oils (for example epoxidized coconut oil, rapeseed oil or soya oil), antifoams, for example silicone oil, preservatives, viscosity regulators, binders and/or tackifiers, fertilizers, in particular nitrogen containing fertilizers such as ammonium nitrates and urea as described in WO08/017388, which can enhance the efficacy of the inventive compounds; or other active ingredients for achieving specific effects, for example ammonium or phosphonium salts, in particular halides, (hydrogen)sulphates, nitrates, (hydrogen)carbonates, citrates, tartrates, formiates and acetates, as described in WO07/068427 and WO07/068428, which also can enhance the efficacy of the inventive compounds and which can be used in combination with penetration enhancers such as alkoxalated fatty acids; bactericides, fungicides, nematocides, plant activators, molluscicides or herbicides.

The compositions according to the invention are prepared in a manner known per se, in the absence of auxiliaries for example by grinding, screening and/or compressing a solid active ingredient and in the presence of at least one auxiliary for example by intimately mixing and/or grinding the active ingredient with the auxiliary (auxiliaries). These processes for the preparation of the compositions and the use of the compounds I for the preparation of these compositions are also a subject of the invention.

The application methods for the compositions, that is the methods of controlling pests of the abovementioned type, such as spraying, atomizing, dusting, brushing on, dressing, scattering or pouring - which are to be selected to suit the intended aims of the prevailing circumstances - and the use of the compositions for controlling pests of the abovementioned type are other subjects of the invention. Typical rates of concentration are between 0.1 and 1000 ppm, preferably between 0.1 and 500 ppm, of active ingredient. The rate of application per hectare is generally 1 to 2000 g of active ingredient per hectare, in particular 10 to 1000 g/ha, preferably 10 to 600 g/ha.

A preferred method of application in the field of crop protection is application to the foliage of the plants (foliar application), it being possible to select frequency and rate of application to match the danger of infestation with the pest in question. Alternatively, the active ingredient can reach the plants via the root system (systemic action), by drenching the locus of the plants with a liquid composition or by incorporating the active ingredient in solid form into the locus of the plants, for example into the soil, for example in the form of granules (soil application). In the case of paddy rice crops, such granules can be metered into the flooded paddy-field.

The compositions according to the invention are also suitable for the protection of plant propagation material, for example seeds, such as fruit, tubers or kernels, or nursery plants, against pests of the abovementioned type. The propagation material can be treated with the compositions prior to planting, for example seed can be treated prior to sowing. Alternatively, the compositions can be applied to seed kernels (coating), either by soaking the kernels in a liquid composition or by applying a layer of a solid composition. It is also possible to apply the compositions when the propagation material is planted to the site of application, for example into the seed furrow during drilling. These treatment methods for plant propagation material and the plant propagation material thus treated are further subjects of the invention.

Further methods of application of the compositions according to the invention comprise drip application onto the soil, dipping of parts of plants such as roots bulbs or tubers, drenching the soil, as well as soil injection. These methods are known in the art.

In order to apply a compound of formula I as an insecticide, acaricide, nematicide or molluscicide to a pest, a locus of pest, or to a plant susceptible to attack by a pest, a compound of formula I is usually formulated into a composition which includes, in addition to the compound of formula I, a suitable inert diluent or carrier and, optionally, a formulation adjuvant in form of a surface active agent (SFA) as described herein or, for example, in EP-B-1062217. SFAs are chemicals which are able to modify the properties of an interface (for example, liquid/solid, liquid/air or liquid/liquid interfaces) by lowering the interfacial tension and thereby leading to changes in other properties (for example dispersion, emulsification and wetting). It is preferred that all compositions (both solid and liquid formulations) comprise, by weight, 0.0001 to 95%, more preferably 1 to 85%, for example 5 to 60%, of a compound of formula I. The composition is generally used for the control of pests such that a compound of formula I is applied at a rate of from 0.1g to10kg per hectare, preferably from 1g to 6kg per hectare, more preferably from 1g to 1 kg per hectare. When used in a seed dressing, a compound of formula I is used at a rate of 0.0001g to 10g (for example 0.001g or 0.05g), preferably 0.005g to 10g, more preferably 0.005g to 4g, per kilogram of seed.

In another aspect the present invention provides an insecticidal, acaricidal, nematicidal or molluscicidal composition comprising an insecticidally, acaricidally, nematicidally or molluscicidally effective amount of a compound of formula I and a suitable carrier or diluent therefor.

In a still further aspect the invention provides a method of combating and controlling pests at a locus which comprises treating the pests or the locus of the pests with an insecticidally, acaricidally, nematicidally or molluscicidally effective amount of a composition comprising a compound of formula I.

The compositions can be chosen from a number of formulation types, including dustable powders (DP), soluble powders (SP), water soluble granules (SG), water dispersible granules (WG), wettable powders (WP), granules (GR) (slow or fast release), soluble concentrates (SL), oil miscible liquids (OL), ultra low volume liquids (UL), emulsifiable concentrates (EC), dispersible concentrates (DC), emulsions (both oil in water (EW) and water in oil (EO)), micro-emulsions (ME), suspension concentrates (SC), oil-based suspension concentrates (OD), aerosols, fogging/smoke formulations, capsule suspensions (CS) and seed treatment formulations. The formulation type chosen in any instance will depend upon the particular purpose en-visaged and the physical, chemical and biological properties of the compound of formula I.

Dustable powders (DP) may be prepared by mixing a compound of formula I with one or more solid diluents (for example natural clays, kaolin, pyrophyllite, bentonite, alumina, montmorillonite, kieselguhr, chalk, diatomaceous earths, calcium phosphates, calcium and magnesium carbonates, sulphur, lime, flours, talc and other organic and inorganic solid carriers) and mechanically grinding the mixture to a fine powder.

Soluble powders (SP) may be prepared by mixing a compound of formula I with one or more water-soluble inorganic salts (such as sodium bicarbonate, sodium carbonate or magnesium sulphate) or one or more water-soluble organic solids (such as a polysaccharide) and, optionally, one or more wetting agents, one or more dispersing agents or a mixture of said agents to improve water dispersibility/solubility. The mixture is then ground to a fine powder. Similar compositions may also be granulated to form water soluble granules (SG).

Wettable powders (WP) may be prepared by mixing a compound of formula I with one or more solid diluents or carriers, one or more wetting agents and, preferably, one or more dispersing agents and, optionally, one or more suspending agents to facilitate the dispersion in liquids. The mixture is then ground to a fine powder. Similar compositions may also be granulated to form water dispersible granules (WG).

Granules (GR) may be formed either by granulating a mixture of a compound of formula I and one or more powdered solid diluents or carriers, or from pre-formed blank granules by absorbing a compound of formula I (or a solution thereof, in a suitable agent) in a porous granular material (such as pumice, attapulgite clays, fuller's earth, kieselguhr, diatomaceous earths or ground corn cobs) or by adsorbing a compound of formula I (or a solution thereof, in a suitable agent) on to a hard core material (such as sands, silicates, mineral carbonates, sulphates or phosphates) and drying if necessary. Agents which are commonly used to aid absorption or adsorption include solvents (such as aliphatic and aromatic petroleum solvents, alcohols, ethers, ketones and esters) and sticking agents (such as polyvinyl acetates, polyvinyl alcohols, dextrins, sugars and vegetable oils). One or more other additives may also be included in granules (for example an emulsifying agent, wetting agent or dispersing agent).

Dispersible Concentrates (DC) may be prepared by dissolving a compound of formula I in water or an organic solvent, such as a ketone, alcohol or glycol ether. These solutions may contain a surface active agent (for example to improve water dilution or prevent crystallisation in a spray tank).

Emulsifiable concentrates (EC) or oil-in-water emulsions (EW) may be prepared by dissolving a compound of formula I in an organic solvent (optionally containing one or more wetting agents, one or more emulsifying agents or a mixture of said agents). Suitable organic solvents for use in ECs include aromatic hydrocarbons (such as alkylbenzenes or alkylnaphthalenes, exemplified by SOLVESSO 100, SOLVESSO 150 and SOLVESSO 200; SOLVESSO is a Registered Trade Mark), ketones (such as cyclohexanone or methylcyclohexanone) and alcohols (such as benzyl alcohol, furfuryl alcohol or butanol), N-alkylpyrrolidones (such as N-methylpyrrolidone or N-octylpyrrolidone), dimethyl amides of fatty acids (such as C₈-C₁₀ fatty acid dimethylamide) and chlorinated hydrocarbons. An EC product may spontaneously emulsify on addition to water, to produce an emulsion with sufficient stability to allow spray application through appropriate equipment. Preparation of an EW involves obtaining a compound of formula I either as a liquid (if it is not a liquid at room temperature, it may be melted at a reasonable temperature, typically below 70°C) or in solution (by dissolving it in an appropriate solvent) and then emulsifiying the resultant liquid or solution into water containing one or more SFAs, under high shear, to produce an emulsion. Suitable solvents for use in EWs include vegetable oils, chlorinated hydrocarbons (such as chlorobenzenes), aromatic solvents (such as alkylbenzenes or alkylnaphthalenes) and other appropriate organic solvents which have a low solubility in water.

Microemulsions (ME) may be prepared by mixing water with a blend of one or more solvents with one or more SFAs, to produce spontaneously a thermodynamically stable isotropic liquid formulation. A compound of formula I is present initially in either the water or the solvent/SFA blend. Suitable solvents for use in MEs include those hereinbefore described for use in in ECs or in EWs. An ME may be either an oil-in-water or a water-in-oil system (which system is present may be determined by conductivity measurements) and may be suitable for mixing water-soluble and oil-soluble pesticides in the same formulation. An ME is suitable for dilution into water, either remaining as a microemulsion or forming a conventional oil-in-water emulsion.

Suspension concentrates (SC) may comprise aqueous or non-aqueous suspensions of finely divided insoluble solid particles of a compound of formula I. SCs may be prepared by ball or bead milling the solid compound of formula I in a suitable medium, optionally with one or more dispersing agents, to produce a fine particle suspension of the compound. One or more wetting agents may be included in the composition and a suspending agent may be included to reduce the rate at which the particles settle. Alternatively, a compound of formula I may be dry milled and added to water, containing agents hereinbefore described, to produce the desired end product.

Oil-based suspension concentrate (OD) may be prepared similarly by suspending finely divided insoluble solid particles of a compound of formula I in an organic fluid (for example at least one mineral oil or vegetable oil). ODs may further comprise at least one penetration promoter (for example an alcohol ethoxylate or a related compound), at least one non-ionic surfactants and/or at least one anionic surfactant, and optionally at least one additive from the group of emulsifiers, foam-inhibiting agents, preservatives, anti-oxidants, dyestuffs, and/or inert filler materials. An OD is intended and suitable for dilution with water before use to produce a spray solution with sufficient stability to allow spray application through appropriate equipment.

Aerosol formulations comprise a compound of formula I and a suitable propellant (for example *n*-butane). A compound of formula I may also be dissolved or dispersed in a suitable medium (for example water or a water miscible liquid, such as n-propanol) to provide compositions for use in non-pressurised, hand-actuated spray pumps.

A compound of formula I may be mixed in the dry state with a pyrotechnic mixture to form a composition suitable for generating, in an enclosed space, a smoke containing the compound.

Capsule suspensions (CS) may be prepared in a manner similar to the preparation of EW formulations but with an additional polymerisation stage such that an aqueous dispersion of oil droplets is obtained, in which each oil droplet is encapsulated by a polymeric shell and contains a compound of formula I and, optionally, a carrier or diluent therefor. The polymeric shell may be produced by either an interfacial polycondensation reaction or by a coacervation procedure. The compositions may provide for controlled release of the compound of formula I and they may be used for seed treatment. A compound of formula I may also be formulated in a biodegradable polymeric matrix to provide a slow, controlled release of the compound.

A composition of the present invention may include one or more additives to improve the biological performance of the composition (for example by improving wetting, retention or distribution on surfaces; resistance to rain on treated surfaces; or uptake or mobility of a compound of formula I). Such additives include surface active agents, spray additives based on oils, for example certain mineral oils or natural plant oils (such as soy bean and rape seed oil), and blends of these with other bio-enhancing adjuvants (ingredients which may aid or modify the action of a compound of formula I).

A compound of formula I may also be formulated for use as a seed treatment, for example as a powder composition, including a powder for dry seed treatment (DS), a water soluble powder (SS) or a water dispersible powder for slurry treatment (WS), or as a liquid composition, including a flowable concentrate (FS), a solution (LS) or a capsule suspension (CS). The preparations of DS, SS, WS, FS and LS compositions are very similar to those of, respectively, DP, SP, WP, SC, OD and DC compositions described above. Compositions for treating seed may include an agent for assisting the adhesion of the composition to the seed (for example a mineral oil or a film-forming barrier).

A composition of the present invention may include one or more additives to improve the biological performance of the composition (for example by improving wetting, retention or distribution on surfaces; resistance to rain on treated surfaces; or uptake or mobility of a compound of formula I). Such additives include surface active agents (SFAs), spray additives based on oils, for example certain mineral oils, vegetable oils or natural plant oils (such as soy bean and rape seed oil), and blends of these with other bio-enhancing adjuvants (ingredients which may aid or modify the action of a compound of formula I). Increasing the effect of a compound of formula I may for example be achieved by adding ammonium and/or phosphonium salts, and/or optionally at least one penetration promotor such as fatty alcohol alkoxylates (for example rape oil methyl ester) or vegetable oil esters.

Wetting agents, dispersing agents and emulsifying agents may be surface active agents (SFAs) of the cationic, anionic, amphoteric or non-ionic type.

Suitable SFAs of the cationic type include quaternary ammonium compounds (for example cetyltrimethyl ammonium bromide), imidazolines and amine salts.

Suitable anionic SFAs include alkali metals salts of fatty acids, salts of aliphatic monoesters of sulphuric acid (for example sodium lauryl sulphate), salts of sulphonated aromatic compounds (for example sodium dodecylbenzenesulphonate, calcium dodecylbenzenesulphonate, butylnaphthalene sulphonate and mixtures of sodium diisopropyl- and tri-isopropyl-naphthalene sulphonates), ether sulphates, alcohol ether sulphates (for example sodium laureth-3-sulphate), ether carboxylates (for example sodium laureth-3-carboxylate), phosphate esters (products from the reaction between one or more fatty alcohols and phosphoric acid (predominately mono-esters) or phosphorus pentoxide (predominately di-esters), for example the reaction between lauryl alcohol and tetraphosphoric acid; additionally these products may be ethoxylated), sulphosuccinamates, paraffin or olefine sulphonates, taurates and lignosulphonates.

Suitable SFAs of the amphoteric type include betaines, propionates and glycinates.

Suitable SFAs of the non-ionic type include condensation products of alkylene oxides, such as ethylene oxide, propylene oxide, butylene oxide or mixtures thereof, with fatty alcohols (such as oleyl alcohol or cetyl alcohol) or with alkylphenols (such as octylphenol, nonylphenol or octylcresol); partial esters derived from long chain fatty acids or hexitol anhydrides; condensation products of said partial esters with ethylene oxide; block polymers (comprising ethylene oxide and propylene oxide); alkanolamides; simple esters (for example fatty acid polyethylene glycol esters); amine oxides (for example lauryl dimethyl amine oxide); and lecithins.

Suitable suspending agents include hydrophilic colloids (such as polysaccharides, polyvinylpyrrolidone or sodium carboxymethylcellulose) and swelling clays (such as bentonite or attapulgite).

A compound of formula I may be applied by any of the known means of applying pesticidal compounds. For example, it may be applied, formulated or unformulated, to the pests or to a locus of the pests (such as a habitat of the pests, or a growing plant liable to infestation by the pests) or to any part of the plant, including the foliage, stems, branches or roots, to the seed before it is planted or to other media in which plants are growing or are to be planted (such as soil surrounding the roots, the soil generally, paddy water or hydroponic culture systems), directly or it may be sprayed on, dusted on, applied by dipping, applied as a cream or paste formulation, applied as a vapour or applied through distribution or incorporation of a composition (such as a granular composition or a composition packed in a water-soluble bag) in soil or an aqueous environment.

A compound of formula I may also be injected into plants or sprayed onto vegetation using electrodynamic spraying techniques or other low volume methods, or applied by land or aerial irrigation systems.

Compositions for use as aqueous preparations (aqueous solutions or dispersions) are generally supplied in the form of a concentrate containing a high proportion of the active ingredient, the concentrate being added to water before use. These concentrates, which may include DCs, SCs, ODs, ECs, EWs, MEs SGs, SPs, WPs, WGs and CSs, are often required to withstand storage for prolonged periods and, after such storage, to be capable of addition to water to form aqueous preparations which remain homogeneous for a sufficient time to enable them to be applied by conventional spray equipment. Such aqueous preparations may contain varying amounts of a compound of formula I (for example 0.0001 to 10%, by weight) depending upon the purpose for which they are to be used.

A compound of formula I may be used in mixtures with fertilisers (for example nitrogen-, potassium- or phosphorus-containing fertilisers, and more particularly ammonium nitrate and/or urea fertilizers). Suitable formulation types include granules of fertiliser. The mixtures suitably contain up to 25% by weight of the compound of formula I.

The invention therefore also provides a fertiliser composition comprising a fertiliser and a compound of formula I.

The compositions of this invention may contain other compounds having biological activity, for example micronutrients or compounds having fungicidal activity or which possess plant growth regulating, herbicidal, safening, insecticidal, nematicidal or acaricidal activity.

The compound of formula I may be the sole active ingredient of the composition or it may be admixed with one or more additional active ingredients such as a pesticide (insect, acarine, mollusc and nematode pesticide), fungicide, synergist, herbicide, safener or plant growth regulator where appropriate. The activity of the compositions according to the invention may thereby be broadened considerably and may have surprising advantages which can also be described, in a wider sense, as synergistic activity. An additional active ingredient may: provide a composition having a broader spectrum of activity or increased persistence at a locus; provide a composition demonstrating better plant/crop tolerance by reducing phytotoxicity; provide a composition controlling insects in their different development stages; synergise the activity or complement the activity (for example by increasing the speed of effect or overcoming repellency) of the compound of formula I; or help to overcome or prevent the development of resistance to individual components. The particular additional active ingredient will depend upon the intended utility of the composition. Examples of suitable pesticides include the following:
a) Pyrethroids, such as permethrin, cypermethrin, fenvalerate, esfenvalerate, deltamethrin, cyhalothrin (in particular lambda-cyhalothrin), bifenthrin, fenpropathrin, cyfluthrin, tefluthrin, fish safe pyrethroids (for example ethofenprox), natural pyrethrin, tetramethrin, s-bioallethrin, fenfluthrin, prallethrin or 5-benzyl-3-furylmethyl-(E)-(1R,3S)-2,2-dimethyl-3-(2-oxothiolan-3-ylidenemethyl)cyclopropane carboxylate;
b) Organophosphates, such as, profenofos, sulprofos, acephate, methyl parathion, azinphos-methyl, demeton-s-methyl, heptenophos, thiometon, fenamiphos, monocrotophos, profenofos, triazophos, methamidophos, dimethoate, phosphamidon, malathion, chlorpyrifos, phosalone, terbufos, fensulfothion, fonofos, phorate, phoxim, pirimiphos-methyl, pirimiphos-ethyl, fenitrothion, fosthiazate or diazinon;
c) Carbamates (including aryl carbamates), such as pirimicarb, triazamate, cloethocarb, carbofuran, furathiocarb, ethiofencarb, aldicarb, thiofurox, carbosulfan, bendiocarb, fenobucarb, propoxur, methomyl or oxamyl;
d) Benzoyl ureas, such as diflubenzuron, triflumuron, hexaflumuron, flufenoxuron or chlorfluazuron;
e) Organic tin compounds, such as cyhexatin, fenbutatin oxide or azocyclotin;
f) Pyrazoles, such as tebufenpyrad and fenpyroximate;
g) Macrolides, such as avermectins or milbemycins, for example abamectin, emamectin benzoate, ivermectin, milbemycin, or spinosad, spinetoram or azadirachtin;
h) Hormones or pheromones;
i) Organochlorine compounds such as endosulfan, benzene hexachloride, DDT, chlordane or dieldrin;
j) Amidines, such as chlordimeform or amitraz;
k) Fumigant agents, such as chloropicrin, dichloropropane, methyl bromide or metam;
l) Neonicotinoid compounds such as imidacloprid, thiacloprid, acetamiprid, clothianidin, nitenpyram, dinotefuran or thiamethoxam;
m) Diacylhydrazines, such as tebufenozide, chromafenozide or methoxyfenozide;
n) Diphenyl ethers, such as diofenolan or pyriproxifen;
o) Indoxacarb;
p) Chlorfenapyr;
q) Pymetrozine or pyrifluquinazon;
r) Spirotetramat, spirodiclofen or spiromesifen;
s) Flubendiamide, chloranthraliniprole, or cyanthraniliprole;
t) Cyenopyrafen or cyflumetofen; or
u) Sulfoxaflor.

In addition to the major chemical classes of pesticide listed above, other pesticides having particular targets may be employed in the composition, if appropriate for the intended utility of the composition. For instance, selective insecticides for particular crops, for example stemborer specific insecticides (such as cartap) or hopper specific insecticides (such as buprofezin) for use in rice may be employed. Alternatively insecticides or acaricides specific for particular insect species/stages may also be included in the compositions (for example acaricidal ovo-larvicides, such as clofentezine, flubenzimine, hexythiazox or tetradifon; acaricidal motilicides, such as dicofol or propargite; acaricides, such as bromopropylate or chlorobenzilate; or growth regulators, such as hydramethylnon, cyromazine, methoprene, chlorfluazuron or diflubenzuron).

In the above-mentioned mixtures of compounds of formula I, in particular a compound selected from said Tables 1 to 36, with other insecticides, fungicides, herbicides, safeners, adjuvants and the like, the mixing ratios can vary over a large range and are, preferably 100:1 to 1:6000, especially 50:1 to 1:50, more especially 20:1 to 1:20, even more especially 10:1 to 1:10. Those mixing ratios are understood to include, on the one hand, ratios by weight and also, on other hand, molar ratios.

The mixtures can advantageously be used in the above-mentioned formulations (in which case "active ingredient" relates to the respective mixture of compound of formula I with the mixing partner).

Some mixtures may comprise active ingredients which have significantly different physical, chemical or biological properties such that they do not easily lend themselves to the same conventional formulation type. In these circumstances other formulation types may be prepared. For example, where one active ingredient is a water insoluble solid and the other a water insoluble liquid, it may nevertheless be possible to disperse each active ingredient in the same continuous aqueous phase by dispersing the solid active ingredient as a suspension (using a preparation analogous to that of an SC) but dispersing the liquid active ingredient as an emulsion (using a preparation analogous to that of an EW). The resultant composition is a suspoemulsion (SE) formulation.

The mixtures comprising a compound of formula I selected from Tables 1 to 36 and one or more active ingredients as described above can be applied, for example, in a single "ready-mix" form, in a combined spray mixture composed from separate formulations of the single active ingredient components, such as a "tank-mix", and in a combined use of the single active ingredients when applied in a sequential manner, i.e. one after the other with a reasonably short period, such as a few hours or days. The order of applying the compounds of formula I selected from Tables 1 to 36 and the active ingredients as described above is not essential for working the present invention.

The invention is illustrated by the following preparation examples. The H-NMR data of certain compounds of this invention show line broadening at room temperature, suggesting the existence of plural conformational isomers due to, for example keto-enol tautomerism, hindered rotation, ring inversion in the piperidine moitey or nitrogen inversion at the piperidine N-OR center. Broad signals have been labeled with 'br' accordingly.

### Example P1: Preparation of 3-(2,5-dimethyl-phenyl)-4-hydroxy-8-methoxy-1,8-diaza-spiro[4.5]dec-3-en-2-one (compound B1)

### Step P1.1: 4-Amino-1-methoxy-piperidine-4-carbonitrile

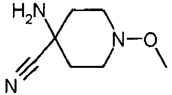

To 10g of N-methoxy-4-piperidone (Journal of Organic Chemistry (1961), 26, 1867-74) in 240ml of ammonium hydroxide (25% in water) was added 6.2g of ammonium chloride and 4.6g of sodium cyanide. After stirring for 18 hours at 25°C the reaction mixture was diluted with 200ml of water and extracted with ethyl acetate. The organic phase was separated, dried over sodium sulfate and evaporated. 8.25g of 4-amino-1-methoxy-piperidine-4-carbonitrile was obtained as a light brown oil, which was used without further purification in the next step.
¹H-NMR (CDCl₃): δ 1.61-2.22 (br signals, total 6H), 2.61-3.43 (br signals, total 4H), 3.51 (s, 3H).

### Step P1.2: 4-Amino-1-methoxy-piperidine-4-carboxylic acid hydrochloride salt

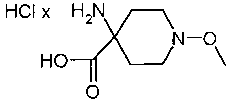

A mixture of 8.25g of 4-amino-1-methoxy-piperidine-4-carbonitrile and 30ml of hydrochloric acid 32% was heated to 100°C. After 16 hours the reacton mixture was evaporated. The solid residue was suspended in ethanol, filtered and dried to obtain 12.5g of 4-amino-1-methoxy-piperidine-4-carboxylic acid as the hydrochloride salt.

### Step P1.3: 4-Amino-1-methoxy-piperidine-4-carboxylic acid methyl ester hydrochloride salt

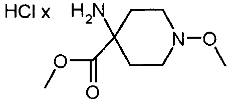

25.7g of thionyl chloride was added at a temperature of 0-10°C within 40 minutes to a suspension of 12.5g of 4-amino-1-methoxy-piperidine-4-carboxylic acid in 100ml of methanol. The reaction mixture was then heated up to 60°C for 48h. After cooling to 20°C, the solids were filtered and the filtrate was evaporated to give 13.2g of 4-amino-1-methoxy-piperidine-4-carboxylic acid methyl ester hydrochloride salt as a brown crystalline solid, mp: 198°C.
¹H-NMR (CDCl₃, free base): δ 1.40-1.72 (br signals, total 2H), 1.58 (s, 2H), 2.02-2.37 (br signals, total 2H), 2.58-2.90 (br signals, total 2H), 3.04-3.32 (br signals, total 2H), 3.52 (s, 3H), 3.73 (s, 3H).
LC-MS (EI, ES+): 189 (M+H)⁺ of the free base.

### Step P1.4: 4-[2-(2,5-dimethyl-phenyl)-acetylamino]-1-methoxy-piperidine-4-carboxylic acid methyl ester

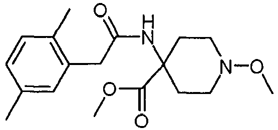

To 5.4g of potassium carbonate and 2g of 4-amino-1-methoxy-piperidine-4-carboxylic acid methyl ester hydrochloride in 10ml of acetonitrile was added 1.94g of (2,5-dimethylphenyl)-acetyl chloride in 5ml of acetonitrile at a temperature of 0-5°C. After stirring for 22 hours at room temperature, the reaction mixture was poured on ice water and extracted with ethyl acetate. The organic phase was dried over sodium sulfate, filtered and evaporated to yield 2.13g of 4-[2-(2,5-dimethyl-phenyl)-acetylamino]-1-methoxy-piperidine-4-carboxylic acid methyl ester as a beige crystalline solid, mp: 91-93°C.
¹H-NMR (CDCl₃): δ 1.95-2.30 (br signals, total 4H), 2.27 (s, 3H), 2.33 (s, 3H), 2.77-3.23 (br signals, total 4H), 3.48 (s, 3H), 3.54 (s, 2H), 3.71 (s, 3H), 5.40 (br s, 1 H), 7.02 (s, 1 H), 7.04 (d, 1H), 7.11 (d, 1 H).
LC-MS (EI, ES+): 335 (M+H)⁺

### Step P1.5: 3-(2,5-dimethyl-phenyl)-4-hydroxy-8-methoxy-1,8-diaza-spiro[4.5]dec-3-en-2-one (compound B1)

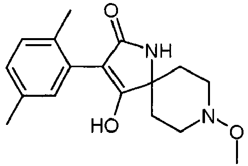

To 0.82g of sodium methylate in 20ml of dimethylformamide was added a solution of 4-[2-(2,5-dimethyl-phenyl)-acetylamino]-1-methoxy-piperidine-4-carboxylic acid methyl ester in 10mol of dimethylformamide at a temperature of 60°C. After stirring for 3 hours at 60°C, the reaction mixture was evaporated. The residue was diluted with 10mol of water, neutralized with 10% hydrochloric acid and extracted with ethyl acetate. The organic phase was dried over sodium sulfate, filtered and evaporated to yield 1.23g of 3-(2,5-dimethyl-phenyl)-4-hydroxy-8-methoxy-1,8-diaza-spiro[4.5]dec-3-en-2-one (title compound B1) as a light brown resin. This material was triturated with diethyl ether/hexane, filtered and dried to afford a solid, mp: 176-177°C.
LC-MS (EI, ES+): 303 (M+H)⁺

### Example P2: Preparation of 8-ethoxy-4-hydroxy-3-(2,4,6-trimethyl-phenyl)-1,8-diaza-spiro[4.5]dec-3-en-2-one (compound B8)

### Step P2.1: 4-Amino-1-ethoxy-piperidine-4-carbonitrile

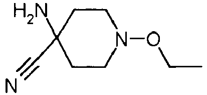

4-Amino-1-ethoxy-piperidine-4-carbonitrile was prepared analogously to the synthesis of 4-amino-1-methoxy-piperidine-4-carbonitrile (preparation example P1, step P1.1) starting from N-ethoxy-4-piperidone (Journal of Organic Chemistry (1961), 26, 1867-74).
¹H-NMR (d₆-DMSO, 88°C): δ 1.08 (t, 3H), 1.71 (m, 2H), 1.93 (m, 2H), 2.38 (br s, 2H), 2.67 (m, 2H), 3.09 (m, 2H), 3.63 (q, 2H).
LC-MS (EI, ES+): 170 (M+H)⁺

### Step P2.2: N-(4-Cyano-1-ethoxy-piperidin-4-yl)-2-(2,4,6-trimethyl-phenyl)-acetamide

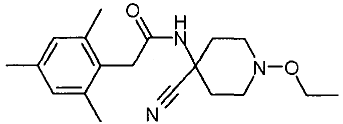

3.0 g of (2,4,6-trimethyl-phenyl)-acetyl chloride and 10 g of potassium carbonate in 90 ml of acetonitrile were treated at 0°C with a solution of 3.6 g of 4-amino-1-ethoxy-piperidine-4-carbonitrile in 30 ml of acetonitrile. After stirring for 18 hours at room temperature the reaction mixture was poured into 100 ml of ice water and extracted with ethyl acetate. The organic phase was washed with brine and concentrated. Chromatography (heptane/ethyl acetate 5:1) yielded 4.5 g of N-(4-cyano-1-ethoxy-piperidin-4-yl)-2-(2,4,6-trimethyl-phenyl)-acetamide as a solid, mp: 194-195°C.
LC-MS (EI, ES+): 330 (M+H)⁺

### Step P2.3: 1-Ethoxy-4-[2-(2,4,6-trimethyl-phenyl)-acetylamino]-piperidine-4-carboxylic acid methyl ester

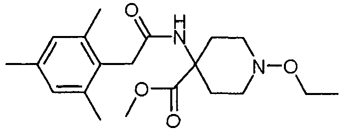

1.4 ml of concentrated sulfuric acid was slowly added to a solution of 4.3 g of N-(4-cyano-1-ethoxy-piperidin-4-yl)-2-(2,4,6-trimethyl-phenyl)-acetamide in 11 ml of methanol. After stirring 20 hours under reflux, the reaction mixture was allowed to cool down to room temperature and diluted with ice water. Sodium carbonate was added and the aqueous phase was extracted with ethyl acetate. The organic phase was dried over sodium sulfate, filtered and concentrated. Chromatography (dichloromethane + 1% of ethanol) gave 3.2 g of 1-ethoxy-4-[2-(2,4,6-trimethyl-phenyl)-acetylamino]-piperidine-4-carboxylic acid methyl ester as a solid, mp: 131-132°C.
LC-MS (EI, ES+): 363 (M+H)⁺

### Step P2.4: 8-Ethoxy-4-hydroxy-3-(2,4,6-trimethyl-phenyl)-1,8-diaza-spiro[4.5]dec-3-en-2-one (compound B8)

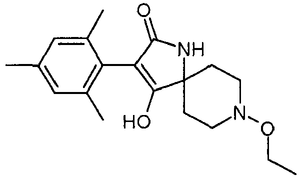

To a solution of 2.3 g of 1-ethoxy-4-[2-(2,4,6-trimethyl-phenyl)-acetylamino]-piperidine-4-carboxylic acid methyl ester in 26 ml of dimethylformamide was added 3 g of sodium methoxide. The reaction mixture was heated to 65°C and stirred for 5 hours. The reaction mixture was poured into brine, neutralized and extracted with ethyl acetate. The organic phase was washed with brine, dried with sodium sulfate, filtered and concentrated. The residue was subjected to column chromatography (heptane/ethyl acetate 2:1) to yield 510 mg of 8-ethoxy-4-hydroxy-3-(2,4,6-trimethyl-phenyl)-1,8-diaza-spiro[4.5]dec-3-en-2-one (title compound B8) as a solid, mp: >250°C.
LC-MS (EI, ES+): 331 (M+H)⁺

### Example P3: Preparation of 4-amino-1-methoxy-piperidine-4-carboxylic acid hydrochloride salt

### Step P3.1: 8-Methoxy-1,3,8-triaza-spiro[4.5]decane-2,4-dione

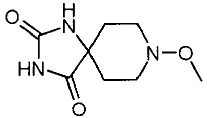

N-methoxy-4-piperidone (Journal of Organic Chemistry (1961), 26, 1867-74) (12.9 g) was added to a solution of ammonium carbonate (14.41 g) and potassium cyanide (13.02 g) in water (200 ml). The reaction mixture was stirred for 30 minutes at room temperature, then for 16 hours at 55-60°C and partially concentrated. The aqueous residue was treated with brine and extracted with ethyl acetate (8x). The combined organic phases were dried over sodium sulfate and concentrated. The crude product was triturated with diethyl ether, filtered and dried. Yield: 11.5 g of 8-methoxy-1,3,8-triaza-spiro[4.5]decane-2,4-dione as a solid, mp: 230-234°C.
¹H-NMR (d₆-DMSO): δ 1.26-1.71 (br signals, total 2H), 1.71-2.12 (br signals, total 2H), 2.50-2.68 and 2.91-3.26 (br signals, total 4H), 3.40 (s, 3H), 8.34 and 8.54 (each br s, total 1 H), 10.64 (br s, 1 H).
LC-MS (EI, ES+): 200 (M+H)⁺

### Step P3.2: 4-Amino-1-methoxy-piperidine-4-carboxylic acid hydrochloride salt

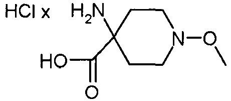

A solution of 8-methoxy-1,3,8-triaza-spiro[4.5]decane-2,4-dione (4.0 g) and sodium hydroxide (0.8 g) in water (25 ml) was heated at 160°C in an autoclave for 18 hours. The reaction mixture was concentrated, the white solid residue taken up in hot methanol, filtered and the filtrate evaporated. The residue was treated with toluol several times to remove water azeotropically until constant weight. Yield: 4.19 g of 4-amino-1-methoxy-piperidine-4-carboxylic acid hydrochloride salt as a solid. This material was identical to the compound described above under preparation example P1, step P1.2.
¹H-NMR (d₄-MeOH): δ 2.13-2.64 (br signals, total 4H), 3.39-3.82 (br signals, total 4H), 3.85 (s, 3H).
LC-MS (EI, ES+): 175 (M+H)⁺ of the free base.

### Example P4: Preparation of 3-(5-cyclopropyl-2,4-dimethyl-phenyl)-4-hydroxy-8-methoxy-1,8-diaza-spiro[4.5]dec-3-en-2-one (compound B16)

### Step P4.1: (5-Cyclopropyl-2,4-dimethyl-phenyl)-acetic acid methyl ester

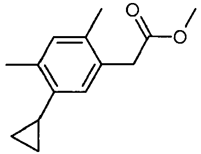

To a solution of 6.0 g of (5-bromo-2,4-dimethyl-phenyl)-acetic acid methyl ester (WO99/48869) in 100 ml of toluene was added 2,2 g of cyclopropylboronic acid and 20 g of potassium phosphate tribasic trihydrate at room temperature. The reaction mixture was stirred for 5 minutes under nitrogen atmosphere, followed by further addition of 1.2 g of tetrakis(triphenylphosphine)palladium(0). After heating and stirring for 16 hours at 110°C, the reaction mixture was filtered, the solvent removed *in vacuo* and the residue was subjected to silica gel chromatography (isohexane/diethyl ether 2:1) to yield 2.5 g of (5-cyclopropyl-2,4-dimethyl-phenyl)-acetic acid methyl ester.

### Step P4.2: (5-Cyclopropyl-2,4-dimethyl-phenyl)-acetic acid

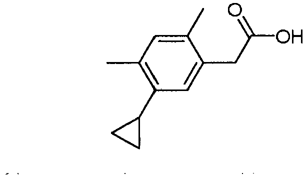

2.5 g of (5-cyclopropyl-2,4-dimethyl-phenyl)-acetic acid methyl ester in 30 ml of methanol was kept at ice bath temperature and treated with 0.5 g of sodium hydroxide in 5 portions. The reaction mixture was stirred at room temperature for 1.5 hours. The solvent was removed *in vacuo,* the residue poured into water and extracted with diethyl ether. The combined organic phases were dried with sodium sulfate, filtered and concentrated to afford 2.3 g of (5-cyclopropyl-2,4-dimethyl-phenyl)-acetic acid.

### Step P4.3: (5-Cyclopropyl-2,4-dimethyl-phenyl)-acetyl chloride

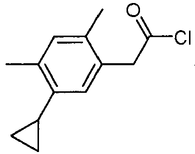

2.3 g of (5-cyclopropyl-2,4-dimethyl-phenyl)-acetic acid in 20 ml of dichloromethane were treated with 2.1 g of oxalyl chloride and a catalytic amount of dimethylformamide. The reaction mixture was stirred at room temperature for 2 hours. The solvent was removed *in vacuo* and the crude residue of (5-cyclopropyl-2,4-dimethyl-phenyl)-acetyl chloride (2.5 g) was used for the next step.

### Step P4.4: 4-[2-(5-Cyclopropyl-2,4-dimethyl-phenyl)-acetylamino]-1-methoxy-piperidine-4-carboxylic acid methyl ester

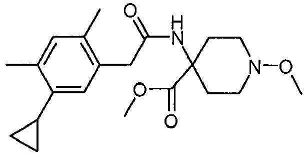

To 4 g of potassium carbonate and 2.5 g of 4-amino-1-methoxy-piperidine-4-carboxylic acid methyl ester hydrochloride (preparation example P1, step P1.3) in 10 ml of acetonitrile was added at 0-5°C a solution of 2.1 g of crude (5-cyclopropyl-2,4-dimethyl-phenyl)-acetyl chloride in 5 ml of acetonitrile. After stirring for 18 hours at room temperature, the solvent was removed *in vacuo* and the crude residue of 4-[2-(5-cyclopropyl-2,4-dimethyl-phenyl)-acetylamino]-1-methoxy-piperidine-4-carboxylic acid methyl ester (3.2 g) was used without further purification in the next step.

### Step P4.5: 3-(5-Cyclopropyl-2,4-dimethyl-phenyl)-4-hydroxy-8-methoxy-1,8-diaza-spiro[4.5]dec-3-en-2-one (compound B16)

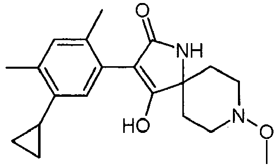

To 1.3 g of sodium methoxide in 10 ml of dimethylformamide was added a solution of 3.2 g of crude 4-[2-(5-cyclopropyl-2,4-dimethyl-phenyl)-acetylamino]-1-methoxy-piperidine-4-carboxylic acid methyl ester in 10 ml of dimethylformamide and the reaction mixture was heated to 65°C for 2 hours. The mixture was poured into ice water, extracted with dichloromethane, the combined organic phases dried with sodium sulfate, filtered and concentrated. The residue was subjected to column chromatography (dichloromethane/methanol 95:5) to yield 700 mg of 3-(5-cyclopropyl-2,4-dimethyl-phenyl)-4-hydroxy-8-methoxy-1,8-diaza-spiro[4.5]dec-3-en-2-one (title compound B16) as a wax.
LC-MS (EI, ES+): 343 (M+H)⁺

### Example P5: Preparation of 3-(5-bromo-4-fluoro-2-methyl-phenyl)-4-hydroxy-8-methoxy-1,8-diaza-spiro[4.5]dec-3-en-2-one (compound B19) and 3-(4'-chloro-6-fluoro-4-methylbiphenyl-3-yl)-4-hydroxy-8-methoxy-1,8-diaza-spiro[4.5]dec-3-en-2-one (compound B24)

### Step P5.1: 1-Bromo-2-fluoro-4-methyl-5-(2,2,2-trichloro-ethyl)-benzene

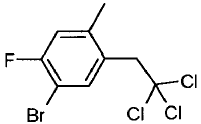

To a solution of vinylidene chloride (127 ml), tert-butyl nitrite (19 ml) and copper(II)chloride (18.4 g) in acetonitrile (150 ml) was added a solution of 5-bromo-4-fluoro-2-methylphenylamine (Bioorganic & Medicinal Chemistry Letters (2006), 16(2), 457-460) (21.5 g) in acetonitrile (100 ml) dropwise below 20°C. The reaction mixture was stirred at room temperature for 48 hours, poured on diluted HCl and extracted with *tert*-butyl methyl ether (3x). The combined organic layers were washed with brine, dried over sodium sulfate and concentrated. The residue was purified by chromatography on silica gel (ethyl acetate/cyclohexane 1:4). Yield: 29.20 g of 1-bromo-2-fluoro-4-methyl-5-(2,2,2-trichloroethyl)-benzene as an oil.
¹H-NMR (CDCl₃): δ 2.42 (s, 3H), 3.93 (s, 2H), 7.00 (d, ³J(H,F)=9.2Hz, 1 H), 7.69 (d, ⁴J(H,F)=7.0Hz, 1H).

### Step P5.2: (5-Bromo-4-fluoro-2-methyl-phenyl)-acetic acid methyl ester

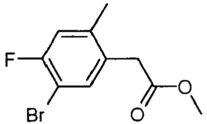

To a solution of 1-bromo-2-fluoro-4-methyl-5-(2,2,2-trichloro-ethyl)-benzene (29.2g) in methanol (100 ml) was added a sodium methoxide solution (30% in methanol, 78.2 ml) dropwise. The reaction mixture was stirred at reflux for 24 hours, cooled to 5°C and treated with concentrated sulfuric acid (13.2 ml) dropwise. After further warming to reflux for 21 hours, the mixture was concentrated and the residue diluted with water/ethyl acetate. The aqueous layer was extracted with ethyl acetate, the combined organic layers were washed with brine, dried over sodium sulfate and concentrated. The residue was purified by chromatography on silica gel (ethyl acetate/cyclohexane 1:3). Yield: 18.76 g of (5-bromo-4-fluoro-2-methyl-phenyl)-acetic acid methyl ester as an oil.
¹H-NMR (CDCl₃): δ 2.24 (s, 3H), 3.56 (s, 2H), 3.69 (s, 3H), 6.93 (d, ³J(H,F)=9.3Hz, 1H), 7.35 (d, ⁴J(H,F)=7.0Hz, 1H).

### Step P5.3: (5-Bromo-4-fluoro-2-methyl-phenyl)-acetic acid

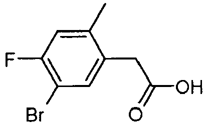

To a solution of (5-bromo-4-fluoro-2-methyl-phenyl)-acetic acid methyl ester (10.3 g) in methanol (50 ml) was added 1 N aqueous sodium hydroxide (47.4 ml) and the reaction mixture was stirred at room temperature for 18 hours. The mixture was concentrated, the residue treated with 1 N hydrochloric acid, the resulting precipitate filtered off, washed with ice-water and dried. Yield: 8.60 g of (5-bromo-4-fluoro-2-methyl-phenyl)-acetic acid as a solid, mp: 100-101°C.
¹H-NMR (CDCl₃): δ 2.26 (s, 3H), 3.60 (s, 2H), 6.95 (d, ³J(H,F)=9.3Hz, 1 H), 7.36 (d, ⁴J(H,F)=7.0Hz, 1 H), 8.6 (br s, 1 H).

### Step P5.4: 4-[2-(5-Bromo-4-fluoro-2-methyl-phenyl)-acetylamino]-1-methoxy-piperidine-4-carboxylic acid methyl ester

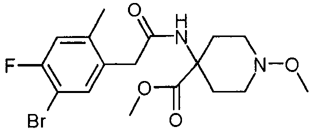

A suspension of (5-bromo-4-fluoro-2-methyl-phenyl)-acetic acid (preparation example P5.3) (8.0 g) and 1,1'-carbonyldiimidazole (5.8 g) in tetrahydrofuran (150 ml) was heated at reflux for 30 minutes. Upon cooling to room temperature, triethylamine (9.0 ml) and 4-amino-1-methoxy-piperidine-4-carboxylic acid methyl ester hydrochloride salt (preparation example P1, step P1.3) (13.8 g) were added and warming at reflux was continued for 3 hours. The cold reaction mixture was poured on water/ethyl acetate, the layers separated, the organic phase washed with brine, dried over sodium sulfate and concentrated. The residue was solubilised in ethyl acetate/cyclohexane 3:1 and purified by filtration on alumina. Yield: 6.83 g of 4-[2-(5-bromo-4-fluoro-2-methyl-phenyl)-acetylamino]-1-methoxy-piperidine-4-carboxylic acid methyl ester as a solid, mp: 192-193°C.
¹H-NMR (CDCl₃): δ 2.04-2.54 (br signals, total 4H), 2.26 (s, 3H), 2.79-3.27 (br signals, total 4H), 3.49 (br s, 5H), 3.71 (s, 3H), 5.40 (br s, 1 H), 6.99 (d, ³J(H,F)=9.3Hz, 1 H), 7.38 (d, ⁴J(H,F)=6.9Hz, 1 H).
MS (FIMS-EI, ES+): 417/419 (M+H)⁺

### Step P5.5: 3-(5-Bromo-4-fluoro-2-methyl-phenyl)-4-hydroxy-8-methoxy-1,8-diaza-spiro[4.5]dec-3-en-2-one (compound B19)

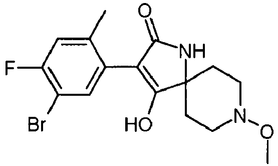

To a solution of 4-[2-(5-bromo-4-fluoro-2-methyl-phenyl)-acetylamino]-1-methoxy-piperidine-4-carboxylic acid methyl ester (6.0 g) in dimethylformamide (20 ml) at 100°C was added potassium *tert*-butoxide (3.23 g) and stirring continued at 100°C for 10 minutes. The reaction mixture was quenched at room temperature by addition of acetic acid (1.64 ml), diluted with water (20 ml) and extracted with *tert*-butyl methyl ether (3x). The combined organic layers were washed with brine, dried over sodium sulfate and concentrated. The residue was triturated with acetonitrile, filtered and dried. Yield: 3.69 g of 3-(5-bromo-4-fluoro-2-methyl-phenyl)-4-hydroxy-8-methoxy-1,8-diaza-spiro[4.5]dec-3-en-2-one (title compound B19) as a solid, mp: 229-230°C.
¹H-NMR (d₆-DMSO): δ 1.45 (m, 2H), 2.09 (s, 3H), 2.15 (m, 2H), 2.62 (m, 2H), 3.25 (m, 2H), 3.42 (s, 3H), 6.93 (d, ³J(H,F)=9.8Hz, 1 H), 7.27 (d, ⁴J(H,F)=7.3Hz, 1 H), 7.92 (br s, 1 H), 10.64 (br s, 1 H).
MS (FIMS-EI, ES-): 383/385 (M-H)⁻

### Step P5.6: 3-(4'-Chloro-6-fluoro-4-methyl-biphenyl-3-yl)-4-hydroxy-8-methoxy-1,8-diaza-spiro[4.5]dec-3-en-2-one (compound B24)

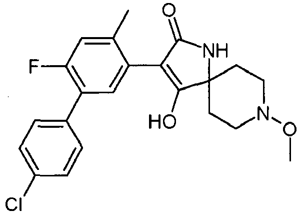

To a suspension of 3-(5-bromo-4-fluoro-2-methyl-phenyl)-4-hydroxy-8-methoxy-1,8-diaza-spiro[4.5]dec-3-en-2-one (230 mg) in dimethoxyethane (10 ml) under nitrogen atmosphere was added tetrakis(triphenylphosphine)palladium(0) (35 mg) and the mixture stirred at room temperature for 15 minutes. After further addition of water (2 ml), 4-chlorophenylboronic acid (112 mg) and sodium carbonate (250 mg), the mixture was heated at reflux for 8 hours. The reaction mixture was acidified at room temperature with 1 N hydrochloric acid and extracted with ethyl acetate (3x). The combined organic layers were washed with brine, dried over sodium sulfate and concentrated. The residue was purified by chromatography on silica gel (ethyl acetate/cyclohexane 5:1). Yield: 170 mg of 3-(4'-chloro-6-fluoro-4-methyl-biphenyl-3-yl)-4-hydroxy-8-methoxy-1,8-diaza-spiro[4.5]dec-3-en-2-one (title compound B24) as a solid.
MS (FIMS-EI, ES+): 417/419 (M+H)⁺; MS (FIMS-EI, ES-): 415/417 (M-H)⁻

### Example P6: Preparation of 3-(5-bromo-3-fluoro-2-methyl-phenyl)-4-hydroxy-8-methoxy-1,8-diaza-spiro[4.5]dec-3-en-2-one (compound C3)

### Step P6.1: 5-Bromo-3-fluoro-2-methyl-phenylamine

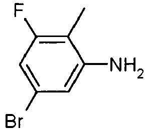

A mixture of 5-bromo-1-fluoro-2-methyl-3-nitro-benzene (9.96 g) and iron powder (11.9 g) in ethanol (100 ml), water (20 ml) and concentrated hydrochloric acid (2 ml) was heated at reflux for one hour. After cooling, the reaction mixture was filtered through hyflo (calcined diatomaceous earth) and concentrated. The residue was purified by chromatography on silica gel (ethyl acetate/cyclohexane 1:9 to 1:4). Yield: 7.06 g of 5-bromo-3-fluoro-2-methylphenylamine as an oil.
¹H-NMR (CDCl₃): δ 1.99 (s, 3H), 3.78 (br s, 2H), 6.60 (s, 1 H), 6.63 (d, ³J(H,F)=9.0Hz, 1 H).

### Step P6.2: (5-Bromo-3-fluoro-2-methyl-phenyl)-acetic acid

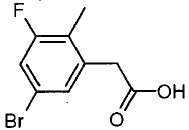

(5-Bromo-3-fluoro-2-methyl-phenyl)-acetic acid was prepared analogously to the synthesis of (5-bromo-4-fluoro-2-methyl-phenyl)-acetic acid (preparation example P5, step P5.3) starting from 5-bromo-3-fluoro-2-methyl-phenylamine (preparation example P6, step P6.1) by making use of the procedures described under step P5.1, step P5.2 and step P5.3. The title acid was obtained as a solid, mp: 147°C.
¹H-NMR (d₆-DMSO): δ 2.07 (d, ⁴J(H,F)=2.1 Hz, 3H), 3.67 (s, 2H), 7.30 (s, 1 H), 7.37 (dd, ³J(H,F)=9.2Hz, J=1.9Hz, 1 H), 12.51 (br s, 1 H).

### Step P6.3: 3-(5-Bromo-3-fluoro-2-methyl-phenyl)-4-hydroxy-8-methoxy-1,8-diaza-spiro[4.5]dec-3-en-2-one (compound C3)

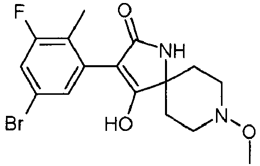

3-(5-Bromo-3-fluoro-2-methyl-phenyl)-4-hydroxy-8-methoxy-1,8-diaza-spiro[4.5]dec-3-en-2-one (title compound C3) was prepared analogously to the synthesis of 3-(5-bromo-4-fluoro-2-methyl-phenyl)-4-hydroxy-8-methoxy-1,8-diaza-spiro[4.5]dec-3-en-2-one (preparation example P5, step P5.5) starting from (5-bromo-3-fluoro-2-methyl-phenyl)-acetic acid (preparation example P6, step P6.2) by making use of the procedures described under step P5.4 and step P5.5. The title compound C3 was obtained as a solid.
¹H-NMR (CDCl₃): δ 1.58 (m, 2H), 1.99 (d, ⁴J(H,F)=2.0Hz, 3H), 2.24 (m, 2H), 2.49 (m, 2H), 3.40 (m, 2H), 3.53 (s, 3H), 6.62 (br s, 1 H), 7.06 (dd, 1 H), 7.07 (s, 1 H).

### Example P7: Preparation of 3-chloro-3-(2,5-dimethyl-phenyl)-8-methoxy-1,8-diaza-spiro[4,5]decane-2,4-dione (compound A1)

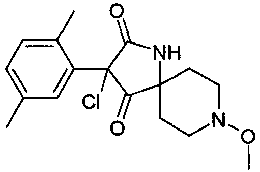

To a solution of 3-(2,5-dimethyl-phenyl)-8-methoxy-1,8-diaza-spiro[4.5]decane-2,4-dione (300 mg, 0.992 mmol) in chloroform (10 ml) at 0°C was added sulfuryl chloride (0.08 ml, 133.2 mg, 0.99 mmol) dropwise. The reaction mixture was stirred at 0°C for one hour, poured on saturated aqueous sodium hydrogen carbonate (5 ml), the layers separated, the water phase extracted twice with dichloromethane, the combined organic phases washed with water and brine, dried over sodium sulfate and concentrated. The residue was triturated in hexane/di-isopropyl ether 4:1, filtered and dried. Yield: 309 mg of 3-chloro-3-(2,5-dimethyl-phenyl)-8-methoxy-1,8-diaza-spiro[4.5]decane-2,4-dione (title compound A1) as a solid, mp 142-144°C.
¹H-NMR (CDCl₃): 1.53-2.00 (br signals, total 2H), 2.21 (br s, 3H), 2.25 (br m, 2H), 2.35 (s, 3H), 2.63 (br m, 2H), 3.14 (br m, 1 H), 3.36 (br m, 1 H), 3.51 (s, 3H), 7.05 (d, 1 H), 7.10 (d, 1 H), 7.54 (s, 1 H), 7.62 and 8.10 (each br s, total 1H).
LC/MS (ES+): 337/339 (M+H)⁺

### Example P8: Preparation of 3-(2,5-dimethyl-phenyl)-3-fluoro-8-methoxy-1,8-diaza-spiro[4.5]decane-2,4-dione (compound A2)

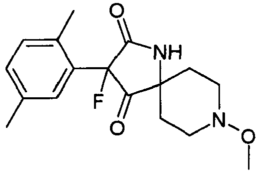

To a solution of 3-(2,5-dimethyl-phenyl)-8-methoxy-1,8-diaza-spiro[4.5]decane-2,4-dione (300 mg, 0.992 mmol) and sodium hydrogen carbonate (110 mg, 1.309 mmol) in acetonitrile (6 ml) at 0°C was added Selectfluor [1-chloromethyl-4-fluoro-1,4-diazonia-bicyclo[2.2.2]octane bis(tetrafluoroborate)] (390 mg, 1.101 mmol) in portions over 10 minutes. The reaction mixture was stirred at 0°C for one hour, at room temperature for one hour, further treated with sodium hydrogen carbonate (11 mg) and selectfluor (39 mg), and stirring continued for 2.5 hours at room temperature. The mixture was diluted with *tert*-butyl methyl ether (5 ml) and aqueous sodium hydrogen carbonate (5 ml), the layers separated, the water phase extracted with *tert*-butyl methyl ether, the combined organic phases washed with brine, dried over sodium sulfate and concentrated. The residue was purified by flash chromatography on silica gel (ethyl acetate/heptane 4:1). Yield: 130 mg of 3-(2,5-dimethyl-phenyl)-3-fluoro-8-methoxy-1,8-diaza-spiro[4.5]decane-2,4-dione (title compound A2) as a solid, mp 127-130°C.
¹H-NMR (CDCl₃): 2.32 (s, 3H), 2.43 (s, 3H), 3.49 (s, 3H), 7.12 (appar s, 3H) [selected signals only].
LC/MS (ES+): 321 (M+H)⁺

### Example P9: Preparation of 3-(4-bromo-2-chloro-6-methyl-phenyl)-3-chloro-8-methoxy-1,8-diaza-spiro[4.5]decane-2,4-dione (compound A7)

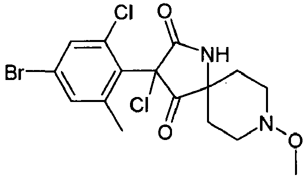

To a solution of 3-(4-bromo-2-chloro-6-methyl-phenyl)-8-methoxy-1,8-diaza-spiro[4.5]decane-2,4-dione (200 mg, 0.498 mmol) and sodium hydrogen carbonate (105 mg, 1.250 mmol) in dichloromethane (5 ml) at -5°C was added sulfuryl chloride (0.038 ml, 63.3 mg, 0.469 mmol) in dichloromethane (0.5 ml) dropwise. The reaction mixture was stirred at 0°C for one hour, poured on saturated aqueous sodium carbonate, the layers were separated, the water phase extracted with ethyl acetate (3x 20 ml), the combined organic phases washed with saturated aqueous sodium carbonate and brine, dried over sodium sulfate and concentrated. The residue was triturated in hexane, filtered and dried. Yield: 190 mg of 3-(4-bromo-2-chloro-6-methyl-phenyl)-3-chloro-8-methoxy-1,8-diaza-spiro[4.5]decane-2,4-dione (title compound A7) as a solid, mp 198°C (dec).
¹H-NMR (CDCl₃): 2.77 (s, 3H), 3.54 (s, 3H), 7.12 (br s, 1 H), 7.37 (s, 2H) [selected signals only].
LC/MS (ES+): 435/437/439/441 (M+H)⁺

### Example P10: Preparation of 8-methoxy-3-nitro-3-(2,4,6-trimethyl-phenyl)-1,8-diaza-spiro[4.5]decane-2,4-dione (compound A16)

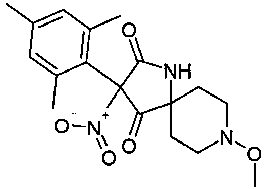

To a solution of 8-methoxy-3-(2,4,6-trimethyl-phenyl)-1,8-diaza-spiro[4.5]decane-2,4-dione (100 mg, 0.316 mmol) in chloroform (10 ml) at -5°C was added fuming nitric acid (0.026 ml, 39.5 mg, 0.627 mmol) in chloroform (0.5 ml) dropwise. The reaction mixture was stirred at 0°C for 30 minutes and at room temperature for one hour, poured on ice water (25 ml), the layers were separated, the water phase extracted with ethyl acetate (3x 20 ml), the combined organic phases washed with water and brine, dried over sodium sulfate and concentrated. The residue was purified by chromatography on silica gel (ethyl acetate). Yield: 80 mg of 8-methoxy-3-nitro-3-(2,4,6-trimethyl-phenyl)-1,8-diaza-spiro[4.5]decane-2,4-dione (title compound A16) as a white solid, mp 136°C (dec).
¹H-NMR (CDCl₃): 2.27 (s, 3H), 2.30 (s, 6H), 3.53 (s, 3H), 6.91 (s, 2H), 7.63 and 7.82 (each br s, total 1 H) [selected signals only].
LC/MS (ES+): 362 (M+H)⁺

Compounds of the Tables A, B and C can be prepared by analogous procedures.

Either one of the following LC-MS methods was used to characterize the compounds:

### Method A

### MS: ZQ Mass Spectrometer from Waters (Single quadrupole mass spectrometer);

lonisation method: Electrospray; Polarity: positive/negative ions; Capillary (kV) 3.00, Cone (V) 30.00, Extractor (V) 2.00, Source Temperature (°C) 100, Desolvation Temperature (°C) 250, Cone Gas Flow (UHr) 50, Desolvation Gas Flow (UHr) 400; Mass range: 150 to 1000 or 100 to 900 Da.

LC: HP 1100 HPLC from Agilent: solvent degasser, quaternary pump (ZCQ) / binary pump (ZDQ), heated column compartment and diode-array detector. Column: Phenomenex Gemini C18, 3 µm particle size, 110 Angström, 30 x 3 mm, Temp: 60°C; DAD Wavelength range (nm): 200 to 500; Solvent gradient: A = water + 0.05% v/v HCOOH, B= Acetonitril/Methanol (4:1, v/v) + 0.04% v/v HCOOH.

| Time (min) | A% | B% | Flow (ml/min) |
|---|---|---|---|
| 0.00 | 95.0 | 5.0 | 1.700 |
| 2.00 | 0.0 | 100.0 | 1.700 |
| 2.80 | 0.0 | 100.0 | 1.700 |
| 2.90 | 95.0 | 5.0 | 1.700 |
| 3.00 | 95.0 | 5.0 | 1.700 |

### Method B

MS: ZMD Mass Spectrometer from Waters (Single quadrupole mass spectrometer) ; lonisation method: Electrospray; Polarity: positive/negative ions; Capillary (kV) 3.80, Cone (V) 30.00, Extractor (V) 3.00, Source Temperature (°C) 150, Desolvation Temperature (°C) 350, Cone Gas Flow (UHr) OFF, Desolvation Gas Flow (UHr) 600; Mass range: 150 to 1000 (100 to 1500 for LowMass) or 100 to 900 Da.

LC: HP 1100 HPLC from Agilent: solvent degasser, binary pump, heated column compartment and diode-array detector. Column: Phenomenex Gemini C18, 3 µm particle size, 110 Angström, 30 x 3 mm, Temp: 60 °C; DAD Wavelength range (nm): 200 to 500; Solvent gradient: A = water + 0.05% v/v HCOOH, B= Acetonitril/Methanol (4:1, v:v) + 0.04% v/v HCOOH.

| Time (min) | A% | B% | Flow (ml/min) |
|---|---|---|---|
| 0.00 | 95.0 | 5.0 | 1.700 |
| 2.00 | 0.0 | 100.0 | 1.700 |
| 2.80 | 0.0 | 100.0 | 1.700 |
| 2.90 | 95.0 | 5.0 | 1.700 |
| 3.00 | 95.0 | 5.0 | 1.700 |

The characteristic values obtained for each compound were the retention time ("Rₜ", recorded in minutes) and the molecular ion as listed in Tables A-C.

**Table A**

| | | | | | | | | |
|---|---|---|---|---|---|---|---|---|
| Compounds of formula la | | | | | | | | |
| 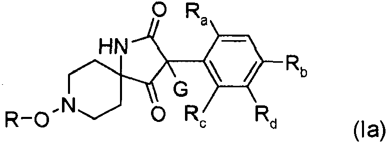 | | | | | | | | |

| | R | G | Rₐ | R_{b} | R_{c} | R_{d} | Mp. | LC-MS |
|---|---|---|---|---|---|---|---|---|
| A1 | CH₃ | Cl | CH₃ | H | H | CH₃ | 142-144°C | 337/339 (M+H)⁺ |
| | | | | | | | | Rₜ = 1.75 min |
| A2 | CH₃ | F | CH₃ | H | H | CH₃ | 127-130°C | 321 (M+H)⁺ |
| | | | | | | | | Rₜ = 1.69 min |
| A3 | CH₃ | Cl | Cl | Cl | H | H | 211-213°C | 375/377/379/381 (M-H)⁻ |
| | | | | | | | | Rₜ = 1.76 min |
| A4 | CH₃ | Cl | CH₃ | H | H | 4-Cl-C₆H₄ | 194-196°C | 433/435/437 (M+H)⁺ |
| | | | | | | | | Rₜ = 2.04 min |
| A5 | CH₃ | Cl | CH₃ | Cl | CH₃ | H | 166-168°C (dec) | 371/373/375 (M+H)⁺ |
| | | | | | | | | Rₜ = 1.87 min |
| A6 | CH₃ | Cl | CH₃ | Br | CH₃ | H | 156°C (dec) | 415/417/419 (M+H)⁺ |
| | | | | | | | | Rₜ = 1.88 min |
| A7 | CH₃ | Cl | Cl | Br | CH₃ | H | 198°C (dec) | 435/437/439/441 (M+H)⁺ |
| | | | | | | | | Rₜ = 1.84 min |
| A8 | CH₃ | Cl | CH₃ | H | CH₃ | H | 165°C (dec) | 337/339 (M+H)⁺ |
| | | | | | | | | Rₜ = 1.70 min |
| A9 | CH₃ | Cl | Cl | CH₃ | CH₃ | H | 183°C (dec) | 371/373/375 (M+H)⁺ |
| | | | | | | | | Rₜ = 1.76 min |
| A10 | CH₃ | Cl | CH₃ | H | CH₃ | Cl | 164°C (dec) | 371/373/375 (M+H)⁺ |
| | | | | | | | | Rₜ = 1.84 min |
| A11 | CH₃ | Cl | CH₃ | Cl | H | CH₃ | 186°C (dec) | 371/373/375 (M+H)⁺ |
| | | | | | | | | Rₜ = 1.85 min |
| A12 | CH₃ | Cl | Cl | CH₃ | H | CH₃ | 205°C (dec) | 371/373/375 (M+H)⁺ |
| | | | | | | | | Rₜ = 1.76 min |
| A13 | CH₃ | Cl | CH₃ | CH₃ | H | CH₃ | 178°C (dec) | 351/353 (M+H)⁺ |
| | | | | | | | | Rₜ = 1.78 min |
| A14 | CH₃ | Cl | CH₃ | CH₃ | CH₃ | H | 173°C (dec) | 351/353 (M+H)⁺ |
| | | | | | | | | Rₜ = 1.79 min |
| A15 | CH₃ | Cl | CH₃ | CH₃ | H | Br | 185°C (dec) | 415/417/419 (M+H)⁺ |
| | | | | | | | | Rₜ = 1.87 min |
| A16 | CH₃ | NO₂ | CH₃ | CH₃ | CH₃ | H | 136°C (dec) | 362 (M+H)⁺ |
| | | | | | | | | Rₜ = 1.83 min |
| A17 | CH₃ | NO₂ | CH₃ | H | H | CH₃ | 145°C (dec) | 348 (M+H)⁺ |
| | | | | | | | | Rₜ = 1.70 min |
| A18 | CH₃ | Cl | Br | CH₃ | Br | H | 174°C (dec) | 479/481/483/485 (M+H)⁺ |
| | | | | | | | | Rₜ = 1.77 min |
| A19 | CH₂C₆H₅ | Cl | CH₃ | CH₃ | CH₃ | H | 165°C (dec) | 427/429 (M+H)⁺ |
| | | | | | | | | Rₜ = 2.11 min |

**Table B**

| | | | | | | |
|---|---|---|---|---|---|---|
| Compounds of formula IIa, wherein cyclo-C3 means cyclopropyl. | | | | | | |
| 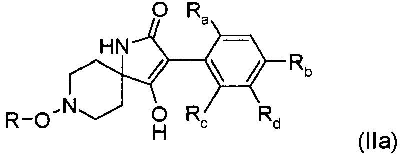 | | | | | | |

| | R | Rₐ | R_{b} | R_{c} | R_{d} | Phys. data |
|---|---|---|---|---|---|---|
| B1 | CH₃ | CH₃ | H | H | CH₃ | 176-177°C |
| B2 | CH₃ | CH₃ | CH₃ | CH₃ | H | 253-255°C |
| B3 | CH₃ | Cl | Cl | H | H | 221-223°C |
| B4 | CH₃ | Cl | H | Cl | H | 257-260°C |
| B5 | CH₃ | CH₃ | H | H | Br | 194-196°C |
| B6 | CH₃ | CH₃ | H | H | 4-Cl-C₆H₄ | solid, LC-MS: 399/401 (M+H)⁺ |
| B7 | CH₃CH₂ | CH₃ | H | H | CH₃ | 166-167°C |
| B8 | CH₃CH₂ | CH₃ | CH₃ | CH₃ | H | >250°C |
| B9 | CH₃CH₂ | Cl | Cl | H | H | 244-245°C |
| B10 | CH₃ | H | Cl | H | Cl | 239-242°C |
| B11 | CH₃ | OCH₃ | H | H | OCH₃ | 194-196°C |
| B12 | CH₃ | CH₃ | Br | CH₃ | H | 260-264°C |
| B13 | CH₃ | CH₃ | cyclo-C3 | CH₃ | H | >250°C |
| B14 | CH₃ | CH₃ | CH₃ | H | Br | solid, LC-MS: 381/383 (M+H)⁺ |
| B15 | CH₃ | CH₃ | CH₃ | H | 4-Cl-C₆H₄ | 168-169°C |
| B16 | CH₃ | CH₃ | CH₃ | H | cyclo-C3 | wax, LC-MS: 343 (M+H)⁺ |
| B17 | CH₃ | Cl | cyclo-C3 | H | H | 206-207°C |
| B18 | CH₃ | CH₃ | cyclo-C3 | H | H | 191-194°C |
| B19 | CH₃ | CH₃ | F | H | Br | 229-230°C |
| B20 | CH₃ | CH₃ | CH₃ | CH₃ | Cl | >200°C |
| B21 | CH₃ | Cl | Br | H | H | 167-169°C |
| B22 | CH₃ | CH₃ | Br | H | H | 187-189°C |
| B23 | CH₃ | CH₃ | 4-Cl-C₆H₄ | CH₃ | H | 255-257°C |
| B24 | CH₃ | CH₃ | F | H | 4-Cl-C₆H₄ | MS: 417/419 (M+H)⁺ |
| B25 | CH₃ | CH₃ | F | H | 4-F-C₆H₄ | |

**Table C**

| | | | | | | |
|---|---|---|---|---|---|---|
| Compounds of formula IIb, wherein cyclo-C3 means cyclopropyl. | | | | | | |
| 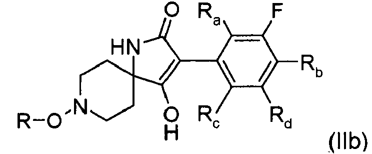 | | | | | | |

| | R | Rₐ | R_{b} | R_{c} | R_{d} | Phys. Data |
|---|---|---|---|---|---|---|
| C1 | CH₃ | CH₃ | H | H | 4-Cl-C₆H₄ | solid |
| C2 | CH₃ | CH₃ | H | H | cyclo-C3 | solid |
| C3 | CH₃ | CH₃ | H | H | Br | solid, 1H-NMR see preparation example P6, step P6.3 |
| C4 | CH₃ | CH₃ | H | H | CH₃ | |
| C5 | CH₃ | CH₃ | H | H | 4-F-C₆H₄ | solid |
| C6 | CH₃CH₂ | CH₃ | H | H | 4-Cl-C₆H₄ | |

Intermediates: compounds of the formula (IV), (V), (VII), (VIII) and (XI) from Tables D, E and F can be prepared by analogous procedures.

**Table D**

| | |
|---|---|
| Compounds of formula IV | |
| 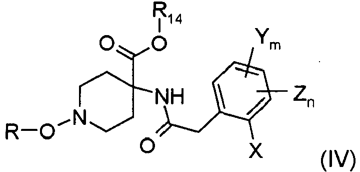 | |

| Structure | Mp. or MS / NMR |
|---|---|
| 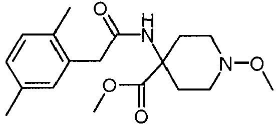 | Preparation example P1, step P1.4 |
| 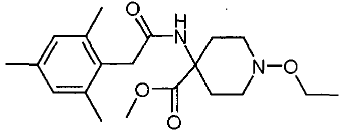 | Preparation example P2, step P2.3 |
| 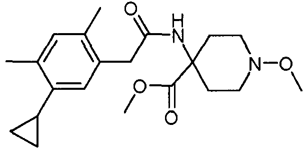 | Preparation example P4, step P4.4 |
| 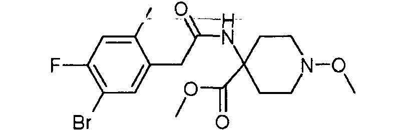 | Preparation example P5, step P5.4 |
| 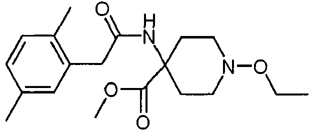 | 132-133°C |
| 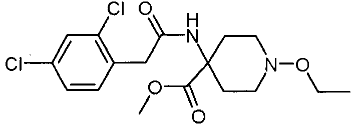 | 166-167°C |

**Table E**

| | |
|---|---|
| Compounds of formula XI | |
| 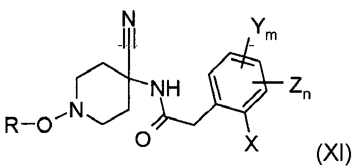 | |

| Structure | Mp. or MS / NMR |
|---|---|
| 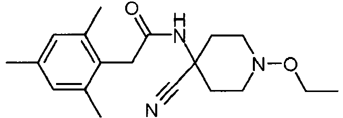 | Preparation example P2, step P2.2 |
| 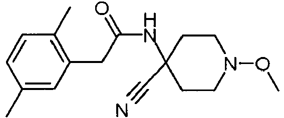 | 208-210°C |
| 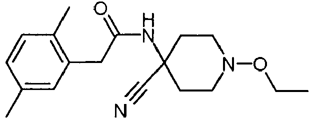 | 159-160°C |
| 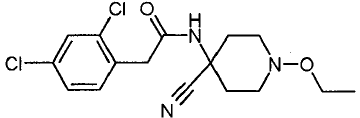 | 169-170°C |

**Table F**

| | |
|---|---|
| Compounds of formula V, VII or VIII | |
| 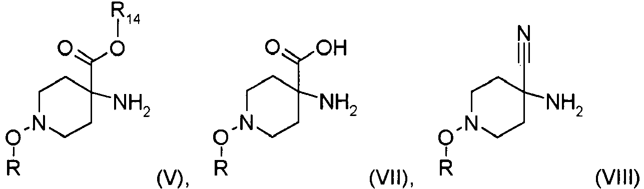 | |

| Structure | Mp. or MS / NMR |
|---|---|
| 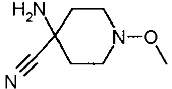 | Preparation example P1, step P1.1 |
| 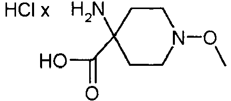 | Preparation example P1, step P1.2 and preparation example P3, step P3.2 |
| 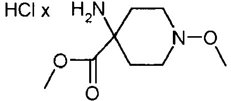 | Preparation example P1, step P1.3 |
| 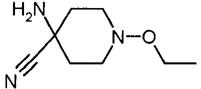 | Preparation example P2, step P2.1 |

### FORMULATION EXAMPLES (% = percent by weight)

| Example F1: Emulsion concentrates | a) | b) | c) |
|---|---|---|---|
| Active ingredient | 25 % | 40 % | 50 % |
| Calcium dodecylbenzenesulfonate | 5 % | 8 % | 6 % |
| Castor oil polyethylene glycol ether (36 mol of EO) | 5 % | - | - |
| Tributylphenoxypolyethylene glycol ether (30 mol of EO) | - | 12 % | 4 % |
| Cyclohexanone | - | 15 % | 20 % |
| Xylene mixture | 65 % | 25 % | 20 % |

Emulsions of any desired concentration can be prepared from such concentrates by dilution with water.

| Example F2: Solutions | a) | b) | c) | d) |
|---|---|---|---|---|
| Active ingredient | 80 % | 10% | 5 % | 95 % |
| Ethylene glycol monomethyl ether | 20 % | - | - | - |
| Polyethylene glycol MW 400 | - | 70% | - | - |
| N-Methylpyrrolid-2-one | - | 20% | - | - |
| Epoxidized coconut oil | - | - | 1 % | 5 % |
| Petroleum ether (boiling range: 160-190°) | - | - | 94 % | - |

The solutions are suitable for use in the form of microdrops.

| Example F3: Granules | a) | b) | c) | d) |
|---|---|---|---|---|
| Active ingredient | 5% | 10% | 8 % | 21 % |
| Kaolin | 94 % | - | 79 % | 54 % |
| Highly disperse silica | 1 % | - | 13 % | 7 % |
| Attapulgite | - | 90% | - | 18 % |

The active ingredient is dissolved in dichloromethane, the solution is sprayed onto the carrier(s), and the solvent is subsequently evaporated in vacuo.

| Example F4: Dusts | a) | b) |
|---|---|---|
| Active ingredient | 2 % | 5 % |
| Highly disperse silica | 1 % | 5 % |
| Talc | 97 % | - |
| Kaolin | - | 90% |

Ready-to-use dusts are obtained by intimately mixing the carriers and the active ingredient.

| Example F5: Wettable powders | a) | b) | c) |
|---|---|---|---|
| Active ingredient | 25 % | 50 % | 75 % |
| Sodium lignosulfonate | 5 % | 5 % | |
| Sodium lauryl sulfate | 3 % | - | 5 % |
| Sodium diisobutylnaphthalenesulfonate | - | 6 % | 10 % |
| Octylphenoxypolyethylene glycol ether (7-8 mol of EO) | - | 2 % | - |
| Highly disperse silica | 5 % | 10 % | 10 % |
| Kaolin | 62 % | 27 % | - |

The active ingredient is mixed with the additives and the mixture is ground thoroughly in a suitable mill. This gives wettable powders, which can be diluted with water to give suspensions of any desired concentration.

| Example F6: Extruder granules | |
|---|---|
| Active ingredient | 10 % |
| Sodium lignosulfonate | 2 % |
| Carboxymethylcellulose | 1 % |
| Kaolin | 87 % |

The active ingredient is mixed with the additives, and the mixture is ground, moistened with water, extruded, granulated and dried in a stream of air.

| Example F7: Coated granules | |
|---|---|
| Active ingredient | 3 % |
| Polyethylene glycol (MW 200) | 3 % |
| Kaolin | 94 % |

In a mixer, the finely ground active ingredient is applied uniformLy to the kaolin, which has been moistened with the polyethylene glycol. This gives dust-free coated granules.

| Example F8a: Suspension concentrate | |
|---|---|
| Active ingredient | 40 % |
| Ethylene glycol | 10 % |
| Nonylphenoxypolyethylene glycol ether (15 mol of EO) | 6 % |
| Sodium lignosulfonate | 10 % |
| Carboxymethylcellulose | 1 % |
| 37 % aqueous formaldehyde solution | 0.2 % |
| Silicone oil (75 % aqueous emulsion) | 0.8 % |
| Water | 32 % |

| Example F8b: Suspension concentrate | |
|---|---|
| Active ingredient | 10% |
| Naphthalenesulfonic acid, sodium salt condensed with formaldehyde | 2% |
| Solution of an acrylic graft copolymer in water and propyleneglycole | 8% |
| Silicone antifoam emulsion | 0.5% |
| DL-propanediol-(1,2) | 3% |
| Heteropolysaccharide | 0.5% |
| 1,2-Benzisothiazol-3-one | 0.2% |
| Water | 75.8% |

The finely ground active ingredient is mixed intimately with the additives. Suspensions of any desired concentration can be prepared from the thus resulting suspension concentrate by dilution with water.

| Example F9: Powders for dry seed treatment | a) | b) | c) |
|---|---|---|---|
| active ingredient | 25 % | 50 % | 75 % |
| light mineral oil | 5 % | 5 % | 5 % |
| highly dispersed silicic acid | 5 % | 5 % | - |
| Kaolin | 65 % | 40 % | - |
| Talcum | - | | 20 % |

The active ingredient is thoroughly mixed with the adjuvants and the mixture is thoroughly ground in a suitable mill, affording powders that can be used directly for seed treatment.

| Example F10: Flowable concentrate for seed treatment | |
|---|---|
| active ingredient | 40 % |
| propylene glycol | 5 % |
| copolymer butanol PO/EO | 2 % |
| tristyrenephenole with 10-20 moles EO | 2 % |
| 1,2-benzisothiazolin-3-one (in the form of a 20% solution in water) | 0.5 % |
| monoazo-pigment calcium salt | 5 % |
| Silicone oil (in the form of a 75 % emulsion in water) | 0.2 % |
| Water | 45.3 % |

The finely ground active ingredient is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

| Example F11a: Oil-based suspension concentrate (based on a vegetable oil) | |
|---|---|
| Active ingredient | 10% |
| Tristyrylphenole with 16 moles EO | 10% |
| Block copolymer of polyhydroxystearic acid and polyalkylene glycols | 2% |
| AEROSIL 200 | 1% |
| Rape seed oil methyl ester | 12% |
| Oleic acid | 65% |

| Example F11b: Oil-based suspension concentrate (based on a mineral oil) | |
|---|---|
| Active ingredient | 10% |
| Ethoxylated alcohols, C16-18 and C18-unsatd | 5% |
| Dodecyl-benzene sulfonic acid Ca-salt linear | 2.5% |
| 2-Pyrrolidinone, 1-ethenylhexadecyl-, homopolymer | 1% |
| Organophilic clay | 1% |
| Mixture of petroleum | 80.5% |

The finely ground active ingredient is mixed intimately with the additives. Suspensions of any desired concentration can be prepared from the thus resulting suspension concentrate by dilution with water.

### BIOLOGICAL EXAMPLES

These examples illustrate the pesticidal/insecticidal properties of compounds of formula I.

### Example B1: Activity against Myzus persicae (green peach aphid)

### (mixed population, feeding/residual contact activity, preventive)

Sunflower leaf discs are placed on agar in a 24-well microtiter plate and sprayed with test solutions. After drying, the leaf discs are infested with an aphid population of mixed ages. After an incubation period of 6 days, samples are checked for mortality and special effects (e.g. phytotoxicity).

In this test, compounds listed in the Table A above show good activity. In particular compounds A1, A2, A3, A4, A5, A6, A7, A8, A9, A10, A11, A12, A13, A14, A15 and A18 show an activity of over 80% at a concentration of 400ppm.

### Example B2: Activity against Myzus persicae (green peach aphid)

### (mixed population, systemic/feeding activity, curative)

Roots of pea seedlings, infested with an aphid population of mixed ages, are placed directly in the test solutions. 6 days after introduction, samples are checked for mortality and special effects on the plant.

In this test, compounds listed in the Table A above show good activity. In particular compounds A1, A3, A5, A7, A8, A9, A12, A13, A14, A15 and A18 show an activity of over 80% at a concentration of 400ppm.

### Example B3: Activity against Tetranychus urticae (two-spotted spider mite)

### (mixed population, feeding/residual contact activity, preventive)

Bean leaf discs on agar in 24-well microtiter plates are sprayed with test solutions. After drying, the leaf discs are infested with mite populations of mixed ages. 8 days later, discs are checked for egg mortality, larval mortality, and adult mortality.

In this test, compounds listed in the Table A above show good activity. In particular compounds A1, A4, A5, A7, A8, A9, A10, A11, A12, A13 and A14 show an activity of over 80% at a concentration of 400ppm.

### Example B4: Activity against Thrips tabaci (onion Thrips)

### (mixed population, feeding/residual contact activity, preventive)

Sunflower leaf discs are placed on agar in a 24-well microtiter plate and sprayed with test solutions After drying, the leaf discs are infested with a thrips population of mixed ages. After an incubation period of 6 days, samples are checked for mortality and special effects (e.g. phytotoxicity).

In this test, compounds listed in the Table A above show good activity. In particular compounds A1, A2, A4, A5, A7, A8, A9, A10, A11, A12, A13, A14 and A15 show an activity of over 80% at a concentration of 400ppm.

### Example B5: Activity against Diabrotica balteata (Corn root worm)

### (larvicide, feeding/residual contact activity, preventive)

24-well microtiter plate (MTP) with artificial diet is treated with test solutions by pipetting. After drying, the MTP's are infested with larvae (L2)(6-10 per well). After an incubation period of 5 days, samples are checked for larval mortality, antifeedant and growth regulation.

In this test, compounds listed in the Table A above show good activity. In particular compounds A1, A4, A11, A12 and A13 show an activity of over 80% at a concentration of 400ppm.

### Example B6: Activity against Frankliniella occidentalis (western flower thrips)

Bean leaf discs on agar in petri dishes or bean plants in a spray chamber are treated with diluted test solutions. After drying leaf discs are cut and placed in plastic cups on the surface of an agar layer and infested with mixed population. 6 days (leaf discs) or 14 days (plants) after the infestation, samples are checked for reduction of treated population and compared to the non treated population.

In this test, compounds listed in the Table A above show good activity. For example compounds A2 and A4 show an activity of over 80% at a concentration of 400ppm.

### Example B7: Activity against Bemisia tabaci (tobacco white fly)

### (larvicide, contact/feeding)

Bean plants are infested with 20-30 adults that were removed after a 4 day egg-laying period. After another 7 days, bean plants with hatched nymphs (N-2) are treated (2 replicates) with the test solutions in a spray chamber. Three weeks later, samples are checked for number of emerged adults. Efficacy was calculeted by comparing number of emerged adults in treated and non treated samples.

In this test, compounds listed in the Table A above show good activity. For example compound A1 shows an activity of over 80% at a concentration of 200ppm.

### Example B8: Activity against Nilaparvata lugens (brown rice planthopper)

### (larvicide, feeding/contact)

Rice seedlings are treated with the diluted test solutions in a spray chamber. After drying, they are infested with 20 N₃ nymphs (2 replicates). 6-12 days after the treatment samples are checked for mortality, growth regulation, and effects on the F₁ generation.

In this test, compounds listed in the Table A above show good activity. For example compounds A1, A2, A4, A7, A8, A9, A13 and A14 show an activity of over 80% at a concentration of 400ppm.

### Example B9: Translaminar activity against Aphis craccivora (cowpea aphid)

French bean leaves (Phaseolus vulgaris) are infested with about 20 mixed age individuals on the lower leaf side using clip cages. 1 day after the infestation, the upper side of the leaves is treated with the test solution by painting. 5 days later, samples are checked for mortality.

In this test, compounds listed in the Table A above show good activity. For example compound A4, A7, A8, A9, A13 and A14 shows an activity of over 80% at a concentration of 400ppm.

### Example B10: Activity against Aonidiella aurantii (red scale)

Treatment of potato tubers by dipping the in the test solution. One day later, tubers are infested with about 50 crawlers. 6-8 weeks after application samples are checked for the number of crawlers of the next generation (compared to the non treated samples).

In this test, compounds listed in the Table A above show good activity. For example compound A1 shows an activity of over 80% at a concentration of 200ppm.

### Example B11: Activity against Aphis gossypii (cotton aphid)

### (mixed population, systemic/feeding)

Roots of pea seedlings, infested with an aphid population of mixed ages, are placed (2 replicates) directly in the test solution. 6 days later, samples are checked for mortality.

In this test, compounds listed in Table A above show good activity. For example compound A1 shows an activity of over 80% at a concentration of 25 ppm.

### Example B12: Activity against Myzus persicae (green peach aphid)

### (mixed population, feeding/residual contact activity, plant damage evaluation)

Pepper plants infested with a mixed population of *Myzus persicae* are treated with diluted test solutions of the compounds in a spray chamber. 6 days after treatment, samples are checked for mortality and for plant damage (phytotoxicity), visual assessment being made using a 0-100% rating scale (100% = total damage to plant; 0% = no damage to plant). In this test, compounds listed in the Table A above show good activity against *Myzus persicae* and acceptable plant compatibility. For example compounds A4, A5, A7, A8 and A13 show an activity of greater or equal to 80% against *Myzus persicae* and damage to pepper plants less or equal to 10% at a concentration of 200ppm.

### Example B13: Insecticide seed treatment

Sugarbeet seeds are treated with test compounds at various rates (mg a.i. /seed). The seeds are sown into pots filled with soil. After placing the seeds on the soil surface they are further covered with soil. Before and during the bioassays, the plants are grown under optimal greenhouse conditions. After 14 days, the plants are infested with cowpea aphid (*Aphis craccivora*) by placing one infested pea seedling containing approximately 150 aphids of a population of mixed developmental stages onto each plant. Seven days after infestation, the number of aphids is counted per plant. Each treatment group is replicated 10 times. Each replicate contains 1 plant.

In this test, compounds listed in Table A above show good activity in the reduction of aphids exposed to sugarbeet plants grown from treated seeds. For example compound A1 shows an efficacy of over 80% in the reduction of aphids compared to the untreated control plants at a rate of 0.1 mg a.i./seed.

### Example B14: Activity against Meloidogyne incognita

### (pouch test, contact)

Cucumber seeds are sown into each pouch which is treated at the same time with test solutions and nematode egg solutions. 12 days after treatment, samples are checked for gall reduction in the root mass. Efficacy was calculated by comparing formation of galls in treated and untreated samples.

In this test, compounds listed in Table A above show good activity. For example compounds A1and A2 show an activity of over 80% at a concentration of 400 ppm.

### Example B15: Activity against Aphis craccivora (cowpea aphid)

### (mixed population, systemic/feeding)

Roots of pea seedlings, infested with an aphid population of mixed ages, are placed (2 replicates) directly in the test solution. 6 days later, samples are checked for mortality. In this test, compounds listed in Table A above show good activity. For example compounds A8, A9, A13 and A14 show an activity of over 80% at a concentration of 25 ppm.

## Claims

1. Compounds of the formula I 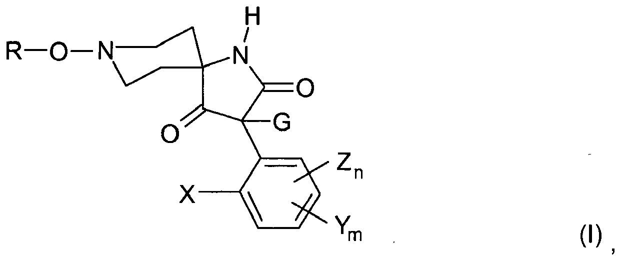 wherein
X , Y and Z, independently of each other, are C₁₋₄alkyl, C₃₋₆cycloalkyl, C₁₋₄haloalkyl, C₁₋₄alkoxy, halogen, phenyl or phenyl substituted by C₁₋₄alkyl, C₁₋₄haloalkyl, halogen or cyano;
m and n, independently of each other, are 0, 1, 2 or 3, where m+n is 0, 1, 2 or 3;
G is halogen or nitro; and
R is hydrogen, C₁₋₆alkyl, C₁₋₆haloalkyl, C₁₋₆cyanoalkyl, C₂-C₆alkenyl, C₃-C₆alkynyl, benzyl, C₁₋₄alkoxy(C₁-₄)alkyl or C₁₋₄alkoxy(C₁₋₄)alkoxy(C₁₋₄)alkyl; or
an agrochemically acceptable salt or an N-oxide thereof.

2. A pesticidal composition comprising a pesticidal effective amount of at least one compound of formula I according to claim 1.

3. A method of combating and controlling pests which comprises applying to a pest or to a plant susceptible to attack by a pest a pesticidally effective amount of a compound of formula I or a pesticidal composition according to claim 2.

4. A process for the preparation of compounds of the formula I according to claim 1, wherein G is halogen or nitro, which comprises reacting compounds of formula II 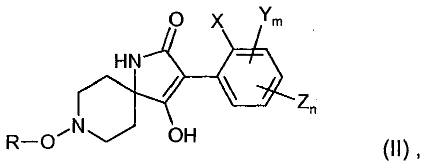 wherein X, Y, Z, m, n and R have the meanings assigned to them in claim 1, with halogenating or nitrating agents, in the presence of a solvent and, optionally, in the presence of a base and/or a free-radical initiator.

5. A pesticidal composition according to claim 2, which, in addition to comprising the compound of formula I, comprises formulation adjuvants.

6. A pesticidal composition according to claim 2, which, in addition to comprising the compound of formula I, comprises at least one additional insecticide, acaricide, nemacitide or molluscicide.

7. A pesticidal composition according to claim 2, which, in addition to comprising the compound of formula I, comprises at least one additional fungicide, herbicide, safener or plant growth regulator.

## Patentansprüche

1. Verbindungen der Formel I 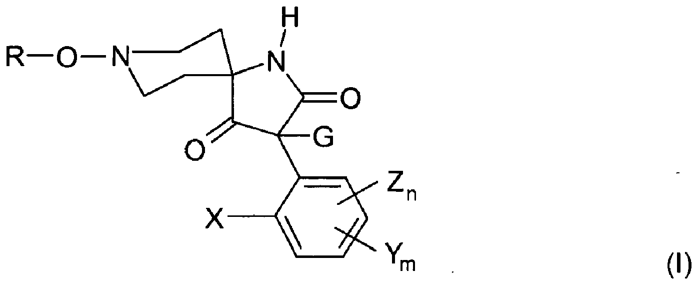 worin
X, Y und Z unabhängig voneinander C₁₋₄-Alkyl, C₃₋₆-Cycloalkyl, C₁₋₄-Halogenalkyl, C₁₋₄-Alkoxy, Halogen, Phenyl oder Phenyl, das durch C₁₋₄-Alkyl, C₁₋₄-Halogenalkyl, Halogen oder Cyano substituiert ist, bedeuten; m und n unabhängig voneinander 0, 1, 2 oder 3 bedeuten, wobei m+n 0, 1, 2 oder 3 ist;
G Halogen oder Nitro bedeutet; und
R Wasserstoff, C₁₋₆-Alkyl, C₁₋₆-Halogenalkyl, C₁₋₆-Cyanoalkyl, C₂₋₆-Alkenyl, C₃₋₆-Alkinyl, Benzyl, C₁₋₄-Alkoxy-(C₁₋₄)-alkyl oder C₁₋₄-Alkoxy-(C₁₋₄)-alkoxy-(C₁₋₄)-alkyl bedeutet; oder
ein agrarchemisch unbedenkliches Salz oder N-Oxid davon.

2. Pestizide Zusammensetzung, die eine pestizidwirksame Menge an mindestens einer Verbindung der Formel I nach Anspruch 1 umfasst.

3. Verfahren zum Bekämpfen und Kontrollieren von Schädlingen, bei dem man eine pestizidwirksame Menge einer Verbindung der Formel I oder eine pestizide Zusammensetzung nach Anspruch 2 auf einen Schädling oder auf eine Pflanze, die gegen Schädlingsbefall anfällig ist, appliziert.

4. Verfahren zur Herstellung von Verbindungen der Formel I nach Anspruch 1, worin G Halogen oder Nitro bedeutet, wobei man bei dem Verfahren Verbindungen der Formel II 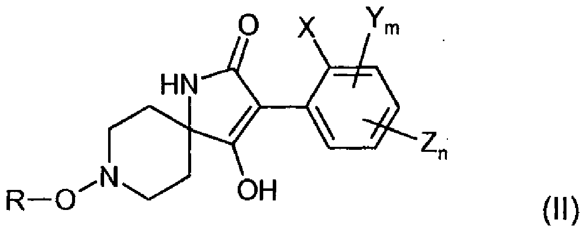 worin X, Y, Z, m, n und R die in Anspruch 1 für sie angegebenen Bedeutungen aufweisen, in Gegenwart eines Lösungsmittels und gegebenenfalls in Gegenwart einer Base und/oder eines Radikalbildners mit Halogenierungsmitteln oder Nitrierungsmitteln umsetzt.

5. Pestizide Zusammensetzung nach Anspruch 2, die nicht nur die Verbindung der Formel I umfasst, sondern auch Formulierungshilfsstoffe umfasst.

6. Pestizide Zusammensetzung nach Anspruch 2, die nicht nur die Verbindung der Formel I umfasst, sondern auch mindestens ein weiteres zusätzliches Insektizid, Akarizid, Nematizid oder Molluskizid umfasst.

7. Pestizide Zusammensetzung nach Anspruch 2, die nicht nur die Verbindung der Formel I umfasst, sondern auch mindestens ein(en) weiteres/n Fungizid, Herbizid, Safener oder Pflanzenwachstumsregulator umfasst.

## Revendications

1. Composés de formule I 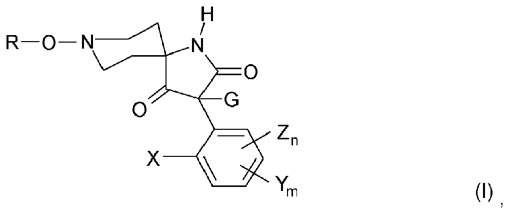 dans laquelle
X, Y et Z, indépendamment l'un de l'autre, sont C₁₋₄-alkyle, C₃₋₆cycloalkyle, C₁₋₄halogénoalkyle, C₁₋₄alcoxy, halogène, phényle ou phényle substitué par C₁₋₄alkyle, C₁₋₄halogénoalkyle, halogène ou cyano ;
m et n, indépendamment l'un de l'autre, valent 0, 1, 2 ou 3, où m+n vaut 0, 1, 2 ou 3 ;
G est halogène ou nitro ; et
R est hydrogène, C₁₋₆alkyle, C₁₋₆halogénoalkyle, C₁₋₆-cyanoalkyle, C₂-C₆alcényle, C₃-C₆alcynyle, benzyle, C₁₋₄-alcoxy(C₁₋₄)alkyle ou C₁₋₄alcoxy(C₁₋₄)alcoxy(C₁₋₄)alkyle; ou
un sel acceptable sur le plan agrochimique ou un N-oxyde de celui-ci.

2. Composition pesticide comprenant une quantité efficace sur le plan pesticide d'au moins un composé de formule I selon la revendication 1.

3. Méthode de lutte et de contrôle de nuisibles, comprenant l'application à un nuisible ou à une plante susceptible d'être attaquée par un nuisible d'une quantité efficace sur le plan pesticide d'un composé de formule I ou d'une composition pesticide selon la revendication 2.

4. Procédé de préparation de composés de formule I selon la revendication 1, **caractérisé en ce que** G est halogène ou nitro, comprenant la réaction de composés de formule II 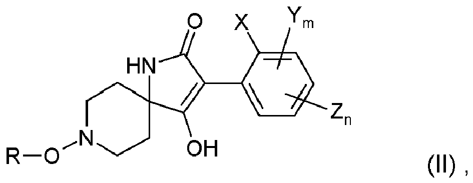 dans laquelle X, Y, Z, m, n et R revêtent les significations leur étant attribuées selon la revendication 1, avec des agents d'halogénation ou de nitratation, en présence d'un solvant et, éventuellement, en présence d'une base et/ou d'un initiateur radicalaire.

5. Composition pesticide selon la revendication 2, qui, outre le composé de formule I, comprend des adjuvants de formulation.

6. Composition pesticide selon la revendication 2, qui, outre le composé de formule I, comprend au moins un autre insecticide, acaricide, nématicide ou molluscicide.

7. Composition pesticide selon la revendication 2, qui, outre le composé de formule I, comprend au moins un(e) autre fongicide, herbicide, agent phytoprotecteur ou substance de croissance.
